(19) Europäisches Patentamt
European Patent Office
Office européen des brevets

(11) Veröffentlichungsnummer: **0 152 379**
**A2**

(12) # EUROPÄISCHE PATENTANMELDUNG

(21) Anmeldenummer: 85810050.6

(22) Anmeldetag: 11.02.85

(51) Int. Cl.⁴: **A 61 K 9/50**

(30) Priorität: 15.02.84 CH 736/84

(43) Veröffentlichungstag der Anmeldung: 21.08.85 Patentblatt 85/34

(84) Benannte Vertragsstaaten: AT BE CH DE FR GB IT LI LU NL SE

(71) Anmelder: CIBA-GEIGY AG
Postfach
CH-4002 Basel(CH)

(72) Erfinder: Muntwyler, René, Dr.
Hutmattweg 6
CH-4114 Hofstetten(CH)

(72) Erfinder: Hauser, Helmut, Dr.
Schwarzbachstrasse 91
CH-8713 Uerikon(CH)

(54) **Verfahren zur Herstellung von pharmazeutischen Zusammensetzungen enthaltend unilamellare Liposomen.**

(57) Die vorliegende Erfindung betrifft ein Verfahren zur Herstellung von pharmazeutischen Zusammensetzungen in Form einer wässrigen wässrigen Dispersion enthaltend unilamellare Liposomen bestehend aus (I) einer amphipatischen Verbindung mit biologischer Wirkung und (II) einem Phospholipid oder einem analogen Lipid und gegebenenfalls einem zusätzlichen Lipid.

Die Liposomendispersion wird durch Herstellung einer homogenen Mischung der Komponenten und Dispersion der homogenen Mischung in wässriger Phase erhalten. Die unilamellaren Liposomen können als Träger von amphipatischen Wirkstoffen unterschiedlichster Art therapeutisch verwendet werden.

EP 0 152 379 A2

Croydon Printing Company Ltd.

0152379

CIBA-GEIGY AG
Basel (Schweiz)

4-14770/+

# Verfahren zur Herstellung von pharmazeutischen Zusammensetzungen enthaltend unilamellare Liposomen

Gegenstand der vorliegenden Erfindung ist ein neues, vorteilhaftes Verfahren zur Herstellung von pharmazeutischen Zusammensetzungen enthaltend unilamellare Liposomen sowie die Verwendung der verfahrensgemäss erhältlichen pharmazeutischen Zusammensetzungen.

Liposomen sind in der Literatur in zahlreichen Veröffentlichungen beschrieben worden. Ihr Aufbau und ihre Verwendung ist Gegenstand vieler Untersuchungen. Man unterscheidet unilamellare Liposomen mit einer Doppelschicht aus Lipiden von multilamellaren Liposomen mit mehreren Doppelschichten aus Lipiden, die zwiebelschalenförmig angeordnet sind.

Unilamellare Liposomen haben einen Durchmesser von ca. $2,0 \times 10^{-8}$ bis $5,0 \times 10^{-6}$m, vorzugsweise ca. $2,0 \times 10^{-8}$ bis $3,0 \times 10^{-6}$m. Die kugelförmige Hülle besteht aus einer Doppelschicht der Lipidkomponenten, z.B. amphipatischen Lipiden, z.B. Phospholipiden, z.B. Lecithin, Kephalin oder Phosphatidsäure, und gegebenenfalls neutralen Lipiden, z.B. Cholesterin. Diese Doppelschicht umschliesst einen Innenraum, der eine wässrige Phase mit einer einzuschliessenden Verbindung enthält, wobei die einzuschliessende Verbindung abhängig von ihrer Struktur und weiteren Parametern wie Temperatur oder Konzentration in der wässrigen Phase und/oder in der Doppelschicht vorhanden sein kann.

Es besteht grosses Interesse an der therapeutischen Verwendung von Liposomen als Träger von Wirkstoffen unterschiedlichster Art. So sind Liposomen als Träger von Proteinen, z.B. Antikörpern oder Enzymen, Hormonen, Vitaminen oder Genen oder zu analytischen Zwecken als Träger von markierten Verbindungen vorgeschlagen worden. Als Beispiel sei die US-Patentschrift 3,993,754 genannt, welche ein chemotherapeutisches Verfahren bei der Behandlung von Tumorzellen unter Verwendung von Liposomen als Träger zum Gegenstand hat.

Kleine unilamellare Liposomen (KUL) mit einem Durchmesser von ca. $2,0 \times 10^{-8}$ bis $1,0 \times 10^{-7}$m eignen sich insbesondere für den Transport durch Barrieren im Organismus, die für grosse Liposomen impermeabel sind, z.B. durch "Fenster" in fenstrierten Kapillaren, Lymphknotengewebe sowie Interstitialräume verschiedener Gewebe.

Der betreffende Wirkstoff wird entweder bei der Bildung der Liposomen oder nachträglich durch Diffusion verkapselt. Die Herstellung von Liposomen und die Verkapselung der Wirkstoffe kann auf verschiedene Weise erfolgen und ist in dem Uebersichtsartikel von Kaye, St.B., Cancer Treatment Reviews (1981) 8, 27-50 beschrieben. Weitere Verfahren zur Herstellung von Liposomen zwecks Verkapselung von Wirkstoffen sind ebenfalls durch Barenholz et al. in Biochemistry, Vol. 16, No. 12, 2806-2810, sowie in den Deutschen Offenlegungsschriften (DOS) 28 19 855, 29 02 672, 25 32 319 und 28 42 608, in der US-Patentschrift 4,053,585 und in der Europäischen Patentanmeldung 36 676 beschrieben.

Nach den bisher bekannt gewordenen Verfahren löst man beispielsweise die Lipidkomponenten, z.B. Phospholipide, z.B. Phosphatidsäure, Lecithin oder Kephalin, und gegebenenfalls neutrale Lipide, z.B. Cholesterin, in einem organischen Lösungsmittel, z.B. Chloroform oder Benzol, auf. Nach dem Eindampfen bleibt eine homogene Schicht, z.B. eine Filmschicht, der betreffenden Lipidkomponenten zurück. Man dispergiert anschliessend die Lipidkomponenten in einer wässrigen

Phase, welche den betreffenden Wirkstoff enthält, z.B. durch Schütteln. Bei der anschliessenden Behandlung mit Ultraschall bilden sich unilamellare Liposomen, welche den Wirkstoff verkapseln.

In der Europäischen Patentanmeldung 88 046 ist ein Verfahren zur Herstellung von unilamellaren Liposomen beschrieben, indem man eine wässrige Dispersion aus zwei verschiedenen Lipiden, z.B. Ei-Phosphatidsäure und Lecithin, und einem Wirkstoff, z.B. einem Muramylpeptid, herstellt und eine Lipidkomponente, z.B. die Phosphatidsäure, in die ionische oder dissoziierte Form durch Aenderung des pH-Werts der wässrigen Phase überführt.

In dem von Gegoriadis G. herausgegebenen Uebersichtswerk "Liposome Technology", CRC-Press 1983, sind in Band II, Kapitel 4, wässrige Lipsomendispersionen beschrieben, welche 8-Aminochinolin-Derivate enthalten. Die Herstellung erfolgt durch Auflösen eines Phospholipids, z.B. Dipalmitoyl- oder Dimyristoylphosphatidylcholin in einem organischen Lösungsmittel, z.B. Chloroform, Herstellung eines Lipidfilms und Dispersion dieses Lipidfilms in wässriger Phase, welche den Wirkstoff, z.B. Primaquin, enthält. Nachteilig ist die auf S. 64 dieser Publikation erwähnte Durchlässigkeit dieser Liposomen, welche Verluste an verkapseltem Wirkstoff verursacht.

Der vorliegenden Erfindung liegt die Aufgabe zugrunde, ein vorteilhaftes und allgemein anwendbaren Verfahren zur Herstellung von pharmazeutischen Zusammensetzungen mit ausreichender Stabilität und hohem Anteil an verkapselten Wirkstoff und unilamellaren Liposomen bereitzustellen.

Diese Aufgabe wird durch die vorliegende Erfindung gelöst, welche ein Verfahren zur Herstellung von pharmazeutischen Zusammensetzungen in Form einer wässrigen Dispersion enthaltend unilamellare Liposomen bestehend aus (I) einer amphipatischen Verbindung mit biologischer Wirkung und (II) einen Phospholipid oder einem analogen Lipid und gegebenenfalls einem zusätzlichen Lipid zum Gegenstand hat.

Das erfindungsgemässe Verfahren ist dadurch gekennzeichnet, dass man (I) die amphipatische Verbindung mit biologischer Wirkung und (II) das Phospholipid oder das analoge Lipid und gegebenenfalls das zusätzliche Lipid homogen mischt und die erhältliche homogene Mischung in wässriger Phase dispergiert und, wenn notwendig, die erhältliche wässrige Dispersion neutralisiert und, wenn erwünscht, die erhältlichen unilamellaren Liposomen anreichert und/oder abtrennt.

Die weiter vorn und im folgenden genannten allgemeinen Begriffe haben im Rahmen der Beschreibung der vorliegenden Erfindung vorzugsweise die folgenden Bedeutungen:

Der im Zusammenhang mit organischen Resten, z.B. Niederalkyl, Niederalkylen, Niederalkoxy, Niederalkanoyl etc., verwendete Ausdruck "Nieder" bedeutet, dass solche organischen Reste, falls nicht ausdrücklich anders definiert, bis einschliesslich 7 und bevorzugt bis einschliesslich 4 Kohlenstoffatome enthalten.

Für die Wirkstoffe werden, wenn nicht anders angegeben, die von der Weltgesundheitsorganisation (WHO) vorgeschlagenen Freinamen (Recommended International Non-proprietary Names) verwendet, welche dem Standardwerk "Pharmazeutische Chemie" (E. Schröder, C. Rufer und R. Schmiechen, Thieme Verlag Stuttgart, 1982), sowie dem Merck-Index (Tenth Edition), entnommen wurden.

Eine amphipatische Verbindung (I) mit biologischer Wirkung, welche mit einem Phospholipid (II) oder einem analogen Lipid und gegebenenfalls einem zusätzlichen Lipid homogen vermischt wird, lässt sich in erster Linie als Arzneimittel verwenden und lässt sich beispielsweise als substituierte Ammonium-Verbindung der Formel

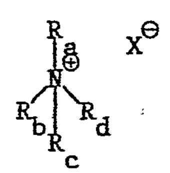

(1),

worin a) $R_a$ eine hydrophobe Gruppe und $R_b$, $R_c$ und $R_d$ unabhängig voneinander Wasserstoff, $C_1$-$C_4$-Alkyl, 2-Hydroxyäthyl, Allyl oder Cyclo-$C_3$-$C_6$-alkyl-$C_1$-$C_3$-alkyl oder zwei der Reste $R_b$, $R_c$ und $R_d$ zusammen gegebenenfalls durch -HN-, -N($C_1$-$C_4$-Alkyl)-, -N(2-Hydroxyäthyl)- oder Sauerstoff unterbrochenes $C_4$- oder $C_5$-Alkylen oder

b) $R_a$ und $R_b$ zwei hydrophobe Gruppen oder zusammen eine hydrophobe Gruppe und $R_c$ und $R_d$ unabhängig voneinander Wasserstoff, $C_1$-$C_4$-Alkyl, Allyl oder Cyclo-$C_3$-$C_6$-alkyl-$C_1$-$C_3$-alkyl oder

c) $R_a$, $R_b$ und $R_c$ zusammen eine hydrophobe Gruppe und $R_d$ Wasserstoff oder $C_1$-$C_4$-Alkyl und $X^\ominus$ das Anion einer pharmazeutisch annehmbaren Säure bedeutet,

als Carbonsäuresalz der Formel

$$R_a\text{-}COO^{\ominus}\ Y^{\oplus} \qquad (2),$$

worin $R_a$ eine hydrophobe Gruppe darstellt und $Y^\oplus$ das Kation einer pharmazeutisch annehmbaren Base ist,

als α-Aminosäure-Verbindung der Formel

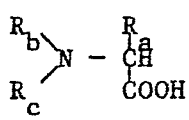

(3),

worin $R_a$ eine hydrophobe Gruppe und $R_b$ und $R_c$ unabhängig voneinander Wasserstoff oder $C_1$-$C_4$-Alkyl darstellen,

als Phosphorsäuremonoester der Formel

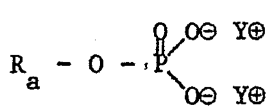

(4),

worin $R_a$ eine hydrophobe Gruppe und Y⊕ das Kation einer pharmazeutisch annehmbaren Base darstellt, oder

als Säureadditionssalz einer Verbindung mit einer hydrophoben Gruppe $R_a$ und einer Imidazolin-, Imidazolidin- oder Hydrazinogruppe als hydrophiler Gruppe klassifizieren.

In einer als Arzneimittel verwendbaren, substituierten Ammonium-Verbindung der Formel 1 ist im Fall a) die hydrophobe Gruppe $R_a$ ein aliphatischer Kohlenwasserstoffrest, der durch ein Sauerstoff- oder Schwefelatom unterbrochen, die Gruppen -C(=O)-, -O-C(=O)-, -C(=O)-NH-, -O-C(=O)-NH- oder Hydroxy enthalten und durch 1-3 gegebenenfalls substituierte, monocyclische, aliphatische oder aromatische Kohlenwasserstoffreste, einen gegebenenfalls substituierten, bi- oder tricyclischen, aromatischen oder partiell gesättigten Kohlenwasserstoffrest, einen gegebenenfalls substituierten, monocyclischen, aromatischen, partiell gesättigten oder gesättigten Heterocyclus oder einen gegebenenfalls substituierten, bi- oder tricyclischen, aromatischen, partiell gesättigten oder Benzo-kondensierten Heterocyclus substituiert sein kann.

Die hydrophobe Gruppe $R_a$ kann auch ein gegebenenfalls substituierter, monocyclischer, aliphatischer oder aromatischer oder ein bicyclischer, aliphatischer oder Benzo-kondensierter Kohlenwasserstoffrest sein. Die hydrophile Gruppe ist beispielsweise eine Gruppe der Formel

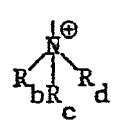

worin $R_b$, $R_c$ und $R_d$ unabhängig voneinander Wasserstoff, $C_1$-$C_4$-Alkyl, z.B. Methyl, Aethyl, Isopropyl oder n-Propyl, oder 2-Hydroxyäthyl oder worin zwei der Reste $R_b$, $R_c$ und $R_d$ zusammen Piperidino, Piperazinyl, 1-Methylpiperazinyl, 1-(2-Hydroxyäthyl)-piperazinyl oder Morpholino und der andere Rest Wasserstoff bedeutet.

In einer als Arzneimittel verwendbaren, substituierten Ammonium-Verbindung der Formel 1 sind im Fall b) $R_a$ und $R_b$ zwei hydrophobe Gruppen, z.B. zwei aliphatische Kohlenwasserstoffreste, welche durch einen oder zwei gegebenenfalls substituierte, monocyclische, aliphatische oder aromatische Kohlenwasserstoffreste oder durch einen gegebenenfalls substituierten, monocyclischen, aromatischen, partiell gesättigten oder gesättigten Heterocyclus substituiert sein können, oder $R_a$ und $R_b$ stellen zusammen einen gegebenenfalls substituierten, monocyclischen, aromatischen, gesättigten, partiell gesättigten oder Benzo-kondensierten Heterocyclus dar. Die hydrophile Gruppe ist eine Gruppe der Formel

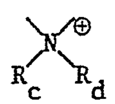

worin $R_c$ und $R_d$ unabhängig voneinander Wasserstoff oder $C_1$-$C_4$-Alkyl, vorzugsweise Methyl, bedeuten.

In einer als Arzneimittel verwendbaren, substituierten Ammonium-Verbindung der Formel 1 bilden im Fall c) $R_a$, $R_b$ und $R_c$ die hydrophobe Gruppe und stellen zusammen einen gegebenenfalls substituierten, aromatischen, partiell gesättigten oder Benzo-kondensierten Heterocyclus dar. Die hydrophile Gruppe ist eine Gruppe der Formel

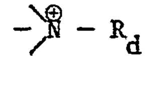

worin $R_d$ Wasserstoff oder $C_1$-$C_4$-Alkyl, vorzugsweise Methyl, bedeutet.

X⊖ ist das Anion einer pharmazeutisch annehmbaren Säure, z.B. einer Mineralsäure, z.B. das Chlorid-, Hydrogensulfat- oder Dihydrogenphosphation, das Bromid- oder Jodidion, oder das Anion einer organischen Säure, z.B. Niederalkancarbonsäure, z.B. das Acetation, einer ungesättigten Carbonsäure, z.B. das Fumarat-, oder Maleination, einer Hydroxysäure, z.B. das Lactat-, Tartrat- oder Citration, oder einer aromatischen Säure, z.B. das Salicylation.

Eine amphipatische, als Arzneimittel verwendbare substituierte Ammonium- Verbindung der Formel 1 oder die entsprechende darin durch Salzbildung überführbaren Aminoverbindung ist beispielsweise eine Verbindung aus der Gruppe der Parasympathomimetica mit quaternären oder tertiären Ammoniumgruppen, z.B. Acetylcholinchlorid, Methacholinchlorid, Carbachol, Muscarin, Pilocarpin oder Arecolin, Cholinesterasehemmer mit zwei tertiären Aminogruppen z.B. Physostigmin, oder mit einer quaternären Ammoniumgruppe, z.B. Neostigminbromid oder Pyridostigminbromid, Neurotransmitter mit einer primären Aminogruppe, z.B. Serotonin- oder Histamin, Serotonin-Antagonisten, worin die hydrophobe Gruppe eine Indol-3-yläthyl-Struktur hat und die hydrophile Gruppe eine primäre oder tertiäre Aminogruppe ist, z.B. Tryptamin, Bufotenin oder Psilocybin, Analgetica vom Morphintyp mit tertiärer Aminogruppe und deren Antagonisten, z.B. der Formel

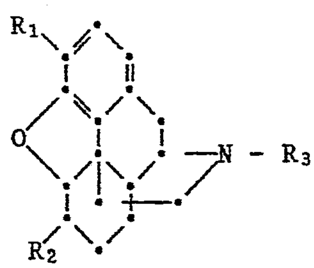

(1.1),

worin $R_1$, $R_2$ und $R_3$ die in der folgenden Liste genannten Bedeutungen haben:

| $R_1$ | $R_2$ | $R_3$ | Name |
|---|---|---|---|
| -OH | -OH | $-CH_3$ | Morphin |
| -OH | =O | $-CH_3$ | Hydromorphon |
| -OH | =O | $-CH_3$ | Oxymorphon |
| -OH | -H | $-CH_3$ | Levorphanol |
| $-OCH_3$ | -OH | $-CH_3$ | Codein |
| $-OCH_3$ | =O | $-CH_3$ | Hydrocodon |
| $-OCH_3$ | =O | $-CH_3$ | Oxycodon |
| -OH | -OH | Allyl | Nalorphin |
| -OH | =O | Allyl | Naloxon |
| -OH | =O | Cyclopropylmethyl | Naltrexon |
| -OH | $-OCH_3$ | Cyclopropylmethyl | Buprenorphin |
| -OH | -H | Cyclobutylmethyl | Butorphanol |
| -OH | -OH | Cyclobutylmethyl | Nalbuphin |
| -2-(Morpholin-1-yl)-äthyl) | -OH | $-CH_3$ | Pholcodin |

Analgetica von Benzomorphan-Typ mit tertiärer Aminogruppe, z.B. Metazocin, Pentazocin oder Cyclazocin, Analgetica vom Pethidin-Typ, z.B. Pethidin, Cetobemidon, Alphaphrodin, Ethoheptazin, Prodilidin oder Profadol, Analgetica des Methadontyps, worin die hydrophobe Gruppe beispielsweise ein 1,1-Diphenyl-1-niederalkyl-2-butanon-Rest und die hydrophile Gruppe Dimethylamino, Morpholino oder Piperidino ist, z.B. Hydrochloride von Verbindungen der Formel

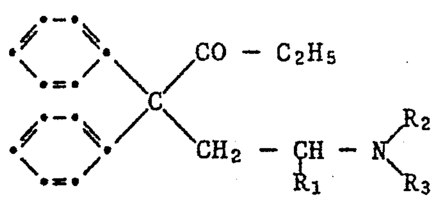

(1.2),

worin $R_1$ Wasserstoff oder Methyl, $R_2$ und $R_3$ Methyl oder $R_2$ und $R_3$ zusammen Morpholino oder Piperidino darstellen, z.B. Methadon, Normethadon, Isomethadon, Dipipanon, Phenadoxon oder Analoga davon

mit Pseudomethadon-Struktur, z.B. Dimepheptanol oder Dextromoramid, Morphin-ähnliche Analgetica mit einer aliphatischen oder cycloaliphatischen tertiären Aminogruppe, z.B. D-Propoxyphen, 1-Benzyl-2-dimethylaminomethyl-1-propanoyloxytetralin, Tramadol, Dimethylthiambuten, Diampromid, Phenampromid, Propiram, Tilidin, Metopholin oder Etonitazen, Analgetica vom Benzimidazol-Typ, z.B. der Formel

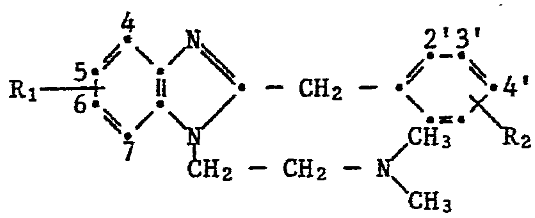

(1.3),

worin $R_1$ 5-, 6- oder 7-Nitro, $R_2$ Wasserstoff, 3'- oder 4'-Methoxy, 4'-Aethoxy, 4'-Isopropoxy, 4'-Methyl oder 4'-Chlor bedeuten, Lokalanästhetica, worin $R_a$ und $R_b$ der Formel 1 zusammen mit dem Stickstoff einen Piperidylrest bilden, der durch eine Niederalkylenbrücke, z.B. 1,3-Propylen, substituiert ist, welche durch eine Methoxycarbonyl- und Benzoyloxy-Gruppe substituiert ist, z.B. Pseudococain oder Cocain, Lokalanästhetica, worin die hydrophobe Gruppe $R_a$ der Formel (1) z.B. die 4-Aminobenzoyloxyäthyl-, 4-Amino-2-chlor-, 2-n-Butoxy- oder 2-Hydroxybenzoyloxyäthyl-, 4-Amino-3-n-butoxybenzoyloxyäthyl-, 3-Amino-4-n-butoxybenzoyloxyäthyl-, 2-Aminobenzoyloxyäthyl-, 2-(4-Aminobenzoyloxy)-6-methyl-n-pentyl-, 4-Aminobenzoyloxy-n-propyl-, 4-n-Butylaminobenzoyloxyäthyl-, 4-n-Butyl-2-hydroxybenzoyl xyäthyl-, 3-(4-n-Propoxybenzoyloxy-2-hydroxy)-propyl-, 2-n-Benzoyloxy-n-propyl-, 2-(2-Acetoxybenzoyloxy)-n-propyl-, Benzoyloxy-n-propyl-, 4-Cyclohexyloxybenzoyloxyäthyl-, 4-Aethyl-oder 4-n-Butylbenzoyloxyäthyl-, 2-n-Butoxychinol-4-ylcarbonyloxyäthyl-, 2,4-Dimethylnilinocarbonylmethyl-, 2-Aethyl-, 2-Chlor- oder 2-Methoxycarbonyl ethyl-4-methylanilinocarbonylmethyl-, 1-(2-Methylnilinocarbonyl)-äthyl-, (2-Aethoxycarbonyl-4-methyl hien-3-ylaminocarbonyl)-äthyl-, 2,3-Dianilinocarbonyloxypropyl-, 4-n-Propyl- oder 4-n-Butylbenzoyläthyl-, 4-Phenoxymethylphenyl-n-butyl-, 4-n-Butoxyphenoxy-n-propyl-, 2-n-Butylchinol-8-yloxymethyl- oder die 8-Benzoyl-

oxycarbonyl-1,2,3,4-tetrahydronaphth-2-yl-Gruppe ist und die hydrophile Gruppe Niederalkylamino, z.B. Methyl-, Aethyl-, Isopropyl-, oder n-Butylamino, Diniederalkylamino, z.B. Dimethyl-, Diäthyl- oder Di-n-propylamino, Cyclohexylamino, 1-Methylpiperid-2-yl, Piperid-1-oder -2-yl oder Morpholin-1-yl ist, z.B. unter den Namen Procain, Chlorprocain, Hydroxyprocain, Propoxycain, Oxybuprocain, Propoxymetacain, Piridocain, Leucinocain, Butacain, Tetracain, Hydroxytetracain, Cornecain, Edan, Piperocain, Cyclomethycain, Parethoxycain, Stadacain, Cinchocain, Lidocain, Pyrrocain, Granocain, Butanilicain,Tolycain, Mepivacain, Bupivacain, Prilocain,Carticain, Dipiperidon, Propicocain, Dyclonin, Pramocain, Fomocain oder Quinisocain bekannt gewordene Lokalanästhetica, Neuroleptica und/oder Thymoleptica, worin die unpolare, hydrophobe Gruppe $R_a$ der Formel 1 ein Niederalkylrest, z.B. Aethyl, n-Propyl, Isoproypl oder n-Butyl, sowie Hydroxyniederalkyl, z.B. 2-Hydroxy-n-propyl, ist, das durch 2-Cyan-, 2-Methoxy-, 2-Chlor-, 2-Trifluormethyl-, 2-Methylthio-, 2-Acetyl- oder 2-Aethyl-10H-phenothiazin-10-yl, 9H-Acridin-10-yl, 5H-Dibenzo[b,f]azepin-5-yl, 7-Chlor-10,11-dihydro-5H-di benzo[b,f] zepin-5-yl, 5,10-Dihydro-5-methyl-11-dibenzo[b,e]-1,4-di azepin-11-onyl, 2-Chlor-, 2-Trifluormethyl- oder 2-Dimethylaminosulfonyl-9H-thioxanthen-9-yliden, 10,11-Dihydro-5H-dibenzo a,d]cyclohepten-5-yl oder 10H-Pyrido[3,2-b]-[1,4]benzothiazin-10-yl substituiert ist und die polare, hydrophile Gruppe Amino, Niederalkylamino, z.B. Methylamino, Diniederalkylamino, z.B. Dimethyl-oder Diäthylamino, Triniederalkylamino, z.B. Trimethyl- oder Triäthylamino, Piperidino oder 4-Hydroxyäthylpiperazino bedeutet, beispielsweise Profenamin, Promethazin, Periciazin, Perimethazin, Chlorpromazin, Perphenazin, Prochlorperazin, Trifluproazin, Trifluorperazin, Fluphenazin, Thioridazin, Mesoridazin, Piperacetazin, Acetophenazin, Ethymemazin, Dimethacrin, Opipramol, Chlomipramin, Imipramin, Desimipramin, Trimipramin, Chlorprotixen, Thiotixen, Amitriptylin, Nortriptylin, Doxepin, Thiepin, Protriptylin oder Prothipendyl, Antidepressiva mit tertiärer Aminogruppe aus der Gruppe bestehend aus Citalopram, Zimelidin, Trebenzomin, Viloxazin, Nomifensin oder Femoxetin, Thymeretica mit einer primären oder durch Methyl und Propargyl substituierten Aminogruppe,

z.B. Tranylcypromin, Pargylin oder Etryptamin, Sedativa, worin die hydrophobe Gruppe $R_a$ der Formel 1 der 2-(7-Chlor-5-o-fluorphenyl-1,3-dihydro-2H-1,4-benzodiazepin-2-on-1-yl)-äthyl-Rest und die hydrophile Gruppe Diäthylamino ist, z.B. Flurazepam, Psychodysleptica mit ß-Phenylethylamin-Struktur, z.B. Mescalin, Psychodysleptica, worin die hydrophobe Gruppe $R_a$ der Formel 1 ein durch einen 3-Indolrest substituierter Aethylrest ist, z.B. $N_\alpha$,$N_\alpha$-Dimethyltryptamin, Bufotenin, Psilocin oder Psilocylein, Psychodysleptica, worin $R_a$ und $R_b$ der Formel 1 zusammen mit dem Stickstoff einen Morpholin oder Pyrrolidinring bilden, der durch 1,3-Niederalkylen substituiert ist, z.B. Scopolamin oder Atropin, Anticholinergica mit Atropinstruktur, z.B. Benzatropin, Anticholinergica (Parkinsonmittel) der Formel

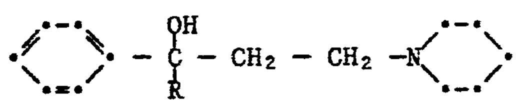

(1.4),

worin R Cyclohexyl, Cyclopentyl, Phenyl oder Norborn-5-en-2-yl bedeutet, z.B. Trihexyphenidyl, Cycrimin, Pridinol oder Biperiden, sowie Analoga wie Procyclidin, Anticholinergica mit einer tertiären Aminogruppe, z.B. Caramiphen, Phenglutarimid, Orphenadrin, Chlorphenoxamin, zentrale Analeptica mit einer Morpholingruppe, z.B. Doxapram, Psychoanaleptica mit Phenylaminopropanstruktur, z.B. Amphetamin, Methamphetamin, Propylhexedrin, Prolintan, Fencamfamin, Methylphenidat, Pipradrol oder Phenmetrazin, Psychoanaleptica mit einer 4-Chlorphenoxyacetoxyäthylgruppe als hydrophober und einer Dimethylaminogruppe als hydrophiler Gruppe, z.B. Meclofenoxat, Vasodilatatoren mit einer tertiären Aminogruppe, z.B. Naftidrofuryl, Appetitzügler mit Amphetamin-Struktur, z.B. Dexamphetamin, Phentermin, Chlorphentermin, Fenfluramin, Amfepramon, Phenmetrazin oder Phendimetrazin, Muskelrelaxantien mit einer hydrophoben und mehreren quartären Aminogruppen, z.B. Tubocumarin, Alcuroniumchlorid, Gallamintriethojodid, Hexcarbacholinbromid, Pancuroniumbromid, Suxamethoniumchlorid oder Decamethoniumbromid, Neurotrope

Spasmolytica mit quartären Aminogruppen, z.B. Scopolaminbutylbromid, Bevoniummethylsulfat, Valethamatbromid oder Methantelinbromid, muskulotrope Spasmolytica mit tertiären Aminogruppen, z.B. Camylofin, Hexahydroadiphenin, Adiphenin oder Fencarbamid, 4-Aminochinolin-Antirheumatica z.B. Chloroquin, Antioestrogene mit tertiärer Aminogruppe, z.B.Tamoxifen oder Ethamoxytriphetol, Histamin-$H_1$-Receptor-Antagonisten (Antihistaminica) mit einer Aethylendiamingruppe, z.B. Phenbenzamin, Tripelenamin, Chlorpyramin, Mepyramin, Metaphenilen, Metapyrilen, Chloropyrilen, Histapyrrodin, Bamipin, Thenalidin, Clemizol, Methdilaazin, Isothipendyl oder Oxomemazin, einer 2-Aminoäthanol-Gruppe, z.B. Diphenhydramin, Medrylamin, Chlorphenoxamin, Silachlorphenoxamin, Carbinoxamin, Diphenpyralin, Clemastin oder Amethobenzepin, oder einer 3-Aminopropan-Gruppe, z.B. Pheniramin, Chlorphenamin, Brompheniramin, Triprolidin, Cycliramin, Phenindamin, Dimetinden, Cyproheptadin oder Ketotifen, Sympathomimetica der Formel

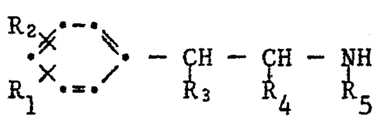

(1.5),

worin $R_1$, $R_2$, $R_3$, $R_4$ und $R_5$ folgende Bedeutungen haben:

| $R_1$ | $R_2$ | $R_3$ | $R_4$ | $R_5$ | Name |
|---|---|---|---|---|---|
| 3-OH | 4-OH | -OH | -H | $-CH_3$ | Epinephrin (Adrenalin) |
| 3-OH | 4-OH | -OH | -H | -H | Norepinephrin (Noradrenalin) |
| 3-OH | 4-OH | -H | -H | -H | Dopamin |
| 3-OH | 4-OH | -OH | $-CH_3$ | -H | Nordefrin |
| 3-OH | 4-OH | -OH | $-C_2H_5$ | -H | Ethylnorepinephrin |
| 3-OH | 4-OH | -OH | -H | $-CH(CH_3)_2$ | Isoprenalin |
| 3-OH | 4-OH | -OH | $-C_2H_5$ | $-CH(CH_3)_2$ | Isoethorin |

| $R_1$ | $R_2$ | $R_3$ | $R_4$ | $R_5$ | Name |
|---|---|---|---|---|---|
| 3-OH | 4-OH | -OH | -H | $-CH(CH_3)_2$ | Metaproterenol |
| 3-OH | 5-OH | -OH | -H | $-C(CH_3)_3$ | Orciprenalin |
| 3-OH | -H | -OH | $-CH_3$ | -H | Metaraminol |
| 3-OH | -H | -OH | -H | $-CH_3$ | Phenylephrin |
| 4-OH | -H | -H | -H | -H | Hydroxyamphetamin |
| $2-OCH_3$ | -H | -H | $-CH_3$ | $-CH_3$ | Methoxyphenamin |
| $2-OCH_3$ | $5-OCH_3$ | -OH | $-CH_3$ | -H | Methoxamin |
| $3-CH_2OH$ | 4-OH | -OH | -H | $-C(CH_3)_3$ | Albuterol |
| -H | -H | -OH | $-CH_3$ | $-CH_3$ | Ephedrin |
| -H | -H | -OH | $-CH_3$ | -H | Norephedrin |
| $3-CF_3$ | -H | -H | $-CH_3$ | $-C_2H_5$ | Fenfluramin |
| -H | -H | -OH | $-CH_3$ | -H | Phenylpropanolamin |
| 4-OH | -H | -OH | $-CH_3$ | $CH_3$ | Pholedrin |
| 4-OH | -H | -OH | $-CH_3$ | -H | Tyramin |
| 3-Cl | 4-Cl | -OH | -H | $-C(CH_3)_3$ | Dichlorisoprenalin |
| 4-OH | -H | -OH | -H | $-CH_3$ | Norfenefrin |
| 4-OH | -H | -OH | -H | -H | Octopamin |
| 3-OH | -H | -OH | -H | $-C_2H_5$ | Etilefrin, |

ß-Receptoren-Blocker der Formel:

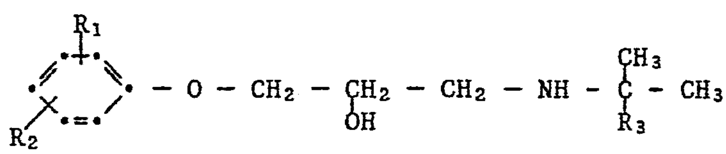

(1.6),

worin $R_1$, $R_2$ und $R_3$ folgende Bedeutungen haben:

| $R_1$ | $R_2$ | $R_3$ | Name |
|---|---|---|---|
| 2-Acetyl | 4-n-Butyrylamino | H | Acebutolol |
| 4-Carbamoylmethyl | H | H | Atenolol |
| 4-(2-Carbamoyläthyl) | H | H | Metoprolol |
| 3-Methyl | H | H | Toliprolol |
| 2-Allyl | H | H | Alprenolol |
| 2-Allyloxy | H | H | Oxprenolol |
| 2-Cyan | H | Methyl | Bunitrolol |
| 2-Chlor | 5-Methyl | Methyl | Bupranolol |
| 3-(N-cyclohexyl-N'-ureido) | H | Methyl | Talinolol |
| 2-Cyclopentyl | H | Methyl | Phenbutolol |
| 2-Tetrahydrofur-2-ylmethoxy | H | Methyl | Bufetolol |
| 2-Pyrrol-1-yl | H | H | |
| 4-(2-Methylthio-äthoxy) | H | H | |
| 4-OH | H | H | Varbian, R,S-Form, S-Form, |

ß-Blocker mit einem bicyclischen, kondensierten Aryloxyrest, z.B. den Naphthyloxy-, Indolyloxy-, 2-Methylindolyloxy-, 1,2,3,4-Tetrahydronaphth-2,3-diol-1-yl- oder 1,2,3,4-Tetrahydronaphth-5-on-1-yl-Rest, z.B. Propanolol, Indenolol, Pindolol, Mepindolol, Nadolol oder Bunolol, sowie ß-Blocker, worin das Segment

$$-O-CH_2-\underset{OH}{\underset{|}{CH}}-NH-$$

 

durch $-O-CH-CH_2-NH-$
OH

ersetzt ist, z.B. Sotalol, Nifenalol, Labetalol oder Bufuralol, Verbindungen mit Wirkung auf periphere Noradrenalinspeicher, z.B. Verbindungen vom Reserpin-Typ, z.B. Reserpin, Rescinnamin oder Syringopin, Tetracyclin-Antibiotica der Formel:

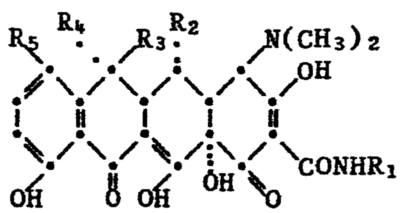

(1.7),

worin $R_1$ Wasserstoff oder Pyrrolidin-1-ylmethyl, $R_2$ Wasserstoff oder Hydroxy, $R_3$ Wasserstoff, Hydroxy oder Methyl, $R_4$ Wasserstoff oder Methyl und $R_5$ Wasserstoff, Chlor oder Dimethylamino bedeuten, z.B. Chlortetracyclin, Oxytetracyclin, Tetracyclin, Demethylchlortetracyclin, Metacyclin, Doxycyclin, Minocyclin oder Rolitetracyclin, Antimalariamittel vom Chinin-Typ, z.B. Conchinidin, Chinidin, Cinchonin, sowie Analoga mit 8-Aminochinolin-Struktur z.B. Pamaquin, Primaquin oder Pentaquin, 4-Aminochinolin-, 9-Aminoacridin-Struktur, z.B. Chloroquin, Santoquin, Hydroxychloroquin, Amodiaquin oder Mepacrin, 1,3,5-Triazin- oder Pyrimidin-Struktur, z.B. Proguanil oder Progianil, Antischistosomatica, worin die hydrophobe, unpolare Gruppe gegebenenfalls 6-Chlor- und/oder 4-Methyl- oder 4-Hydroxymethyl-substituiertes Xanthonyl oder Thioxanthonyl ist und die hydrophile, polare Gruppe Diäthylamino ist, z.B. Lucanthon, Hycanthon, Miracil A oder B, antivirale Mittel des Typs cyclische Amine, z.B. Amantadin, Cyclooctylamin oder Rimantadin, sowie Glucocorticoide, die in 21-Stellung mit einer Aminosäure verestert sind, z.B. Prednisolon-diäthylaminoacetat.

In einem als Arzneimittel verwendbaren Carbonsäuresalz der Formel 2 ist die hydrophobe Gruppe $R_a$ ein aliphatischer Kohlenwasserstoffrest, der durch einen gegebenenfalls substituierten, monocyclischen, aromatischen Kohlenwasserstoffrest oder einen gegebenenfalls substituierten, bi- oder tricyclischen, aromatischen oder partiell gesättigten Kohlenwaserstoffrest, einen gegebenenfalls substituierten, monocyclischen, aromatischen oder partiell gesättigten Heterocyclus oder einen gegebenenfalls substituierten, bi- oder tricyclischen, aromatischen, partiell gesättigten oder Benzo-kondensierten Heterocyclus oder einen Steroidrest substituiert sein kann, oder ist ein gegebenenfalls substituierter, monocyclischer, aromatischer Kohlenwasserstoffrest, ein gegebenenfalls substituierter, bi- oder tricyclischer, aromatischer oder partiell gesättigter Kohlenwasserstoffrest, ein gegebenenfalls substituierter, monocyclischer, aromatischer oder partiell gesättigter Heterocyclus oder ein gegebenenfalls substituierter, bi- oder tricyclischer, aromatischer, partiell gesättigter oder Benzo-kondensierter Heterocyclus.

Das Kation $Y^{\oplus}$ einer pharmazeutisch annehmbaren Base ist beispielsweise ein Alkalimetall-, z.B. Lithium-, Natrium- oder Kaliumion, ein Erdalkalimetall-, z.B. Magnesium- oder Calciumion, sowie das Ammonium-, Mono-, Di- oder Tri-$C_1$-$C_4$-alkylammoniumion, z.B. Trimethyl-, Aethyl-, Diäthyl- oder Triäthylammonium, 2-Hydroxyäthyl-tri-$C_1$-$C_4$-alkylammoniumion, z.B. Cholinyl, sowie das Kation einer basischen Aminosäure, z.B. Lysin oder Arginin.

Carbonsäuresalze der Formel 2 mit biologischer Wirkung oder darin durch Salzbildung überführbare Carbonsäuren sind z.B. Salze von Glucocorticoiden, die in 21-Stellung mit einer Dicarbonsäure verestert sind, z.B. Methylprednisolon-natriumsuccinat, Prednisolonnatriumsuccinat, Kurznarcotica vom 3,20-Dioxo-5ß-pregnan-Typ, welche durch Bernsteinsäure verestert sein können, z.B. Hydroxydionsuccinat-Natrium oder 11,20-Dioxo-3α-hydroxy-5α-pregnan, z.B. Alphaxolon, oder die 21-Verbindung, z.B. Alphadolon, Salze von Choleritica, z.B. Cholsäure- oder Desoxycholsäuresalze, Analgetica, z.B. Salze von substituierten Phenylessigsäuren oder 2-Phenylpropionsäuren, z.B. Alclofenac, Ibufenac, Ibuprofen, Clindanac, Fenclorac, Ketoprofen, Fenoprofen, Indoprofen, Fenclofenac, Diclofenac, Flurbiprofen, Pirprofen, Naproxan, Benoxaprofen, Carprofen oder Cicloprofen, anagetisch wirksame Anthranilsäure-Derivate, z.B. der Formel

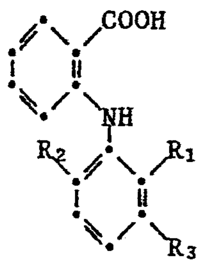

(2.1),

worin $R_1$, $R_2$ und $R_3$ unabhängig voneinander Wasserstoff, Methyl, Chlor oder Trifluormethyl bedeuten, z.B. Mefenaminsäure, Flufenaminsäure, Tolfenaminsäure oder Meclofenaminsäure, analgetisch wirksame Anilino-substituierte Nicotinsäure-Derivate, z.B. Mifluminsäure, Clonixin oder Flunixin, analgetisch wirksame Heteroarylessigsäuren oder 2-Heteroarylpropionsäuren mit einem 2-Indol-3-yl- oder Pyrrol-2-yl-Rest, z.B. Indometacin, Oxmetacin, Intrazol, Acemetazin, Cinmetacin, Zomepirac, Tolmetin, Colpirac oder Tiaprofensäure, analgetisch wirksame Indenylessigsäuren, z.B. Sulindac, analgetisch wirksame Heteroaryloxyessigsäuren, z.B. Benzadac, Prostansäuren, welche die glatte Muskulatur stimulieren, z.B. $PGE_2$ (Dinoproston), $PGF_{2\alpha}$ (Dinoprost), 15 (S)-15-Methyl-$PGE_2$, 15 (S)-15-Methyl-$PGF_{2\alpha}$ (Carboprost), (±)15 (Xi)-15-Methyl-13,14-dihydro-11-desoxy-$PGE_1$ (Deprostil), 15 (S)-15-Methyl-11-desoxy-$PGE_1$ (Doxaprost), 16,16-Dimethyl-$PGE_2$, 17-Phenyl-18,19,20-trinor-$PGF_{2\alpha}$, 16-Phenoxy-17,18,19,20-tetranor-$PGF_{2\alpha}$, z.B. Cloprostenol oder Fluprostenol, oder N-Methylsulfonyl-16-phenoxy-17,18,19,20-tetranor-$PGF_{2\alpha}$ (Sulproston), Bakteriostatica, z.B. Salze von Nalixidinsäurederivaten, z.B. Nalixidinsäure, Cinoxacin, Oxolinsäure, Pironidsäure oder Pipenidsäure, Penicillansäure- und Cephalosporansäurederivate mit antibiotischer Wirkung mit 6ß- bzw. 7ß-Acylaminogruppen, welche in fermentativ, halb- oder totalsynthetisch

erhältlichen 6ß-Acylaminopenicillansäure- oder 7ß-Acylaminocephalosporansäurederivaten oder in 3-Stellung abgewandelten 7ß-Acylaminocephalosporansäurederivaten vorhanden sind, z.B. unter den Namen Penicillin G oder V, Phenethicillin, Propicillin, Nafcillin, Oxacillin, Cloxacillin, Dicloxacillin, Flucloxacillin, Cyclacillin, Epicillin, Mecillinam, Methicillin, Azlocillin, Sulbenicillin, Ticarcillin, Mezlocillin, Piperacillin, Carindacillin, Azidocillin oder Ciclazillin bekannt gewordene Penicillansäurederivate oder unter den Namen Cefaclor, Cefuroxim, Cefazlur, Cephacetril, Cefazolin, Cephalexin, Cefadroxil, Cephaloglycin, Cefoxitin, Cephaloridin, Cefsulodin, Cefotiam, Ceftazidin, Cefonicid, Cefotaxim, Cefmenoxim, Ceftizoxim, Cephalothin, Cephradin, Cefamandol, Cephanon, Cephapirin, Cefroxadin, Cefatrizin, Cefazedon, Ceftrixon oder Ceforanid bekannt gewordene Cephalosporinderivate, sowie andere ß-Lactam-Antibiotica, z.B. Moxalactam, Clavulansäure, Nocardicin A, Sulbactam, Aztreonam oder Thienamycin, oder Antineoplastica mit 4-[Bis-(2-chlorethyl)-aminophenyl]-buttersäure-Struktur, z.B. Chlorambucil oder Antineoplastica mit zwei Carboxygruppen, z.B. Methotrexat.

Verbindungen der Formel 3 mit biologischer Wirkung sind z.B. Neurotransmitter, worin die hydrophobe Gruppe durch Hydroxyphenyl substituiertes Methyl ist, z.B. L-Tyrosin, L-Dopa, α-Methyldopa oder Metirosin, Schilddrüsenhormone mit Jod-substituierten Phenylresten, z.B. Levothyrosin, Diiodotyrosin oder Liothyronin oder Antineoplastica mit Aminosäurestruktur, z.B. Melphalen.

In einer Verbindung der Formel 4 mit biologischer Wirkung ist die unpolare, hydrophobe Gruppe $R_a$ ein Glucocorticoid-Rest und X⊕ Natrium, z.B. Betamethasondinatriumphosphat, Dexamethasondinatriumphosphat, Cortisonphosphat, Hydrocortisonphosphat, Prednisolon-dinatriumphosphat oder Paramethason-21-dinatriumphosphat.

Salzartige Verbindungen mit einer hydrophoben Gruppe und einer Imidazolin-, Imidazolidin- oder Hydrazinogruppe als hydrophiler Gruppe sind beispielsweise Salze von antidepressiv wirksamen Hydrazinderivaten, z.B. Iproniazid, Nialamid, Isocarboxazid, Phenelzin, Pheniprazin, Mebanazin oder Fenoxypropazin, α-Sympathomimetica mit Imidazolin-Struktur, z.B. Naphazolin, Tetryzolin, Tramazolin, Xylometazolin oder Oxymetazolin, α-Sympatholytica mit Imidazolin-Struktur, z.B. Phentolamin oder Tolazolin oder zentral wirkende Antihypertensiva mit Imidazolin-Struktur, z.B. Clonidin, Tolonidin oder Flutonidin oder Vasodilatatoren mit einer Hydrazino-Gruppe, z.B. Dihydralazin, Hydralazin oder Picodralazin.

Ein Phospholipid (II), welches mit der amphipatischen Verbindung (I) mit biologischer Wirkung homogen vermischt wird, hat beispielsweise die Formel

$$R_1-CH_2-\overset{R_3}{\underset{R_2}{C}}-CH_2-O-\overset{O}{\underset{OH}{P}}-O-R_4 \qquad (5),$$

worin einer der Reste $R_1$ und $R_2$ Wasserstoff, Hydroxy, $C_1$-$C_4$-Alkyl und der andere Rest Alkyl, Alkenyl, Alkoxy, Alkenyloxy oder Acyloxy mit je 10 bis 20 C-Atomen oder beide Reste $R_1$ und $R_2$ Alkyl, Alkenyl, Alkoxy, Alkenyloxy oder Acyloxy mit je 10 bis 20 C-Atomen, $R_3$ Wasserstoff oder $C_1$-$C_4$-Alkyl, und $R_4$ Wasserstoff, gegebenenfalls substituiertes $C_1$-$C_7$-Alkyl oder einen Kohlehydratrest mit 5 bis 12 C-Atomen oder, wenn beide Reste $R_1$ und $R_2$ Wasserstoff oder Hydroxy bedeuten, einen Steroidrest bedeutet, oder ist ein Salz davon.

In einem Phospholipid der Formel 5 ist $R_1$, $R_2$ oder $R_3$ mit der Bedeutung $C_1$-$C_4$-Alkyl bevorzugt Methyl, ferner Aethyl, n-Propyl oder n-Butyl.

Alkyl $R_1$ oder $R_2$ ist vorzugsweise geradkettig mit einer geraden Anzahl von 10 bis 20 C-Atomen, z.B. n-Decyl, n-Dodecyl (Lauryl), n-Tetradecyl (Myristyl), n-Hexadecyl (Cetyl), n-Octadecyl (Stearyl) oder n-Eicosyl (Arachinyl).

Alkenyl $R_1$ und/oder $R_2$ ist vorzugsweise geradkettig mit einer geraden Anzahl von 12 bis 20 C-Atomen und einer Doppelbindung, z.B. 9-cis-Dodecenyl (Lauroleyl), 9-cis-Tetradecenyl (Myristoleyl), 9-cis-Hexadecenyl (Palmitoleinyl), 6-cis-Octadecenyl (Petroselinyl) 6-trans-Octadecenyl (Petroselaidinyl), 9-cis-Octadecenyl (Oleyl), 9-trans-Octadecenyl (Elaidinyl) oder 9-cis-Eicosenyl (Gadoleinyl).

Alkoxy $R_1$ und/oder $R_2$ ist vorzugsweise geradkettig mit einer geraden Anzahl von 10 bis 20 C-Atomen, z.B. n-Decyloxy, n-Dodecyloxy (Lauryloxy), n-Tetradecyloxy (Myristyloxy), n-Hexadecyloxy (Cetyloxy), n-Octadecyloxy (Stearyloxy) und n-Eicosyloxy (Arachinyloxy).

Alkenyloxy $R_1$ und/oder $R_2$ ist vorzugsweise geradkettig mit einer geraden Anzahl von 12 bis 20 C-Atomen, z.B. 9-cis-Dodecenyloxy (Lauroleyloxy), 9-cis-Tetradecenyloxy (Myristoleyloxy), 9-cis-Hexadecenyloxy (Palmitoleinyloxy), 6-cis-Octadecenyloxy (Petroselinyloxy), 6-trans-Octadecenyloxy (Petroselaidinyloxy), 9-cis-Octadecenyloxy (Oleyloxy), 9-trans-Octadecenyloxy (Elaidinyloxy) und 9-cis-Eicosenyl (Gadoleinyloxy).

Acyloxy $R_1$ und/oder $R_2$ ist vorzugsweise geradkettig mit einer geraden Anzahl von 10 bis 20 C-Atomen, z.B. Alkanoyloxy oder Alkenoyloxy, vorzugsweise n-Decanoyloxy, n-Dodecanoyloxy (Lauroyloxy), n-Tetradecanoyloxy (Myristoyloxy), n-Hexadecanoyloxy (Palmitoyloxy), n-Octadecanoyloxy (Stearoyloxy) und n-Eicosoyloxy (Arachinoyloxy).

Alkenoyloxy $R_1$ und/oder $R_2$ ist vorzugsweise geradkettig mit einer geraden Anzahl von 10 bis 20 C-Atomen, z.B. 9-cis-Dodecenyloxy (Lauroleoyloxy), 9-cis-Tetradecenoyloxy (Myristoleoyloxy), 9-cis-Hexadecenoyloxy (Palmitoleinoyloxy), 6-cis-Octadecenoyloxy (Petroselinoyloxy), 6-trans-Octadecenoyloxy (Petroselaidinoyloxy), 9-cis-Octadecenoyloxy (Oleoyloxy), 9-trans-Octadecenoyloxy (Elaidinoyloxy) und 9-cis-Eicosenoyloxy (Gadoleinoyloxy).

Gegebenenfalls substituiertes $C_1$-$C_7$-Alkyl $R_4$ ist z.B. Methyl, Aethyl, Isopropyl, n-Propyl, Isobutyl oder n-Butyl, welches durch saure Gruppen, z.B. Carboxy oder Sulfo, saure und basische Gruppen, z.B. Carboxy und Amino, wobei die Aminogruppe sich in α-Stellung zur Carboxygruppe befindet, freie oder verätherte Hydroxygruppen, wobei zwei verätherte Hydroxygruppen durch einen bivalenten Kohlenwasserstoffrest, z.B. durch Methylen, Aethylen, Aethyliden, 1,2-Propylen oder 2,2-Propylen, miteinander verbunden sein können, Halogen, z.B. Chlor oder Brom, Niederalkoxycarbonyl, z.B. Methoxy- oder Aethoxycarbonyl, oder durch Niederalkansulfonyl, z.B. Methansulfonyl, substituiert sein kann.

Substituiertes $C_1$-$C_7$-Alkyl $R_4$ ist beispielsweise Carboxyniederalkyl, z.B. Carboxymethyl, 2-Carboxyäthyl oder 3-Carboxy-n-propyl, ω-Amino-ω-carboxyniederalkyl, z.B. 2-Amino-2-carboxyäthyl oder 3-Amino-3-carboxy-n-propyl, Hydroxyniederalkyl, z.B. 2-Hydroxyäthyl oder 2,3-Dihydroxypropyl, Niederalkoxyniederalkyl, z.B. Methoxy- oder Aethoxymethyl, 2-Methoxyäthyl oder 3-Methoxy-n-propyl, Niederalkylendioxyniederalkyl, z.B. 2,3-Aethylendioxypropyl oder 2,3-(2,2-Propylen)-dioxypropyl, oder Halogenniederalkyl, z.B. Chlor oder Brommethyl, 2-Chlor- oder 2-Bromäthyl, 2- oder 3-Chlor- oder 2-oder 3-Brom-n-propyl.

Substituiertes $C_1$-$C_7$-Alkyl $R_4$ ist vorzugsweise durch Triniederalkylammonium z.B. Trimethyl- oder Triäthylammonium, oder Amino substituiertes Aethyl, z.B. 2-Trimethylammoniumäthyl, oder 2-Ammoniumäthyl, oder ω-Amino-ω-carboxyniederalkyl, z.B. 2-Amino-2-carboxyäthyl.

Ein Kohlehydratrest $R_4$ mit 5 bis 12 C-Atomen ist beispielsweise ein natürlicher Monosaccharidrest, der sich von einer als Aldose oder Ketose vorliegenden Pentose oder Hexose ableitet.

Eine als Aldose vorliegende Pentose ist z.B. D-Ribose, D-Arabinose, D-Xylose oder D-Lyxose.

Eine als Ketose vorliegende Pentose ist z.B. D-Ribulose oder D-Xylulose.

Eine als Aldose vorliegende Hexose ist z.B. D-Allose, D-Altrose, D-Glucose, D-Mannose, D-Galactose oder D-Talose.

Eine als Ketose vorliegende Hexose ist z.B. D-Psicose, D-Fructose, D-Sorbose oder D-Tagatose.

Eine Hexose liegt vorzugsweise in zyklischer Form vor, z.B. als Pyranose (Aldose), z.B. α- oder ß-D-Fructose. Der Pyranosylrest ist vorzugsweise durch die in 1- oder 6-Stellung und der Furanosylrest durch in 1- oder 5-Stellung befindliche Hydroxygruppe mit der Phosphatidylgruppe verestert.

Ein Kohlehydratrest $R_4$ mit 5 bis 12 C-Atomen ist ferner ein natürlicher Disaccharidrest, z.B. ein aus zwei Hexosen gebildeter Disaccaridrest, der sich beispielsweise durch Kondensation von zwei Aldosen, z.B. D-Glucose oder D-Galactose, oder einer Aldose, z.B. D-Glucose mit einer Ketose, z.B. Fructose, bildet. Aus zwei Aldosen gebildete Disaccharide, z.B. Lactose oder Maltose, sind vorzugsweise über die in 6-Stellung des betreffenden Pyranosylrests befindliche Hydroxygruppe mit der Phosphatidylgruppe verestert. Aus einer Aldose und einer Ketose gebildete Disaccharide, z.B. Saccharose, sind vorzugsweise über die in 6-Stellung des Pyranosylrests oder über die in 1-Stellung des Furanosylrests befindliche Hydroxygruppe mit der Phosphatidylgruppe verestert.

Ein Kohlehydratrest $R_4$ mit 5 bis 12 C-Atomen ist ferner ein derivatisierter Mono- oder Disaccharidrest, worin beispielsweise die Aldehydgruppe und/oder ein oder zwei endständige Hydroxygruppen zu Carboxygruppen oxydiert sind, z.B. D-Glucon-, D-Glucar- oder

D-Glucoronsäurereste, welche vorzugsweise als zyklische Lactonreste vorliegen. Ebenso können in einem derivatisierten Mono- oder Disaccharidrest Aldehyd- oder Ketogruppen zu Hydroxygruppen reduziert sein, z.B. Inosit, Sorbit oder D-Mannit, oder Hydroxygruppen durch Wasserstoff, z.B. Desoxyzucker, z.B. 2-Desoxy-D-ribose, L-Rhamnose oder L-Fucose, oder durch Aminogruppen, z.B. Aminozucker, z.B. D-Glucosamin oder D-Galactosamin, ersetzt sein.

Ein Kohlehydratrest $R_4$ kann ebenfalls ein durch Umsetzung eines der genannten Mono- oder Disaccharide mit einem starken Oxydationsmittel, z.B. Perjodsäure, gebildetes Spaltprodukt sein.

Ein Steroidrest $R_4$ ist beispielsweise ein Sterinrest, der über die in 3-Stellung des Steroidgerüsts befindliche Hydroxygruppe mit der Phosphatidylgruppe verestert ist.

Ein Sterinrest ist beispielsweise der Lanosterin-, Sitosterin-, Koprostanol-, Cholestanol-, Glycocholsäure-, Ergosterin- oder Stigmasterinrest, vorzugsweise der Cholesterinrest.

Wenn $R_4$ einen Steroidrest darstellt, sind $R_1$ und $R_2$ vorzugsweise Hydroxy und $R_3$ ist Wasserstoff.

Phospholipide der Formel 5 können in Form von freien Säuren oder als Salze vorliegen. Salze werden durch Umsetzung der freien Säure der Formel II mit einer Base, z.B. einer verdünnten, wässrigen Lösung eines Alkalimetallhydroxides z.B. Lithium-, Natrium- oder Kaliumhydroxid, von Magnesium- oder Calciumhydroxid, einer verdünnten wässrigen Ammoniaklösung oder einer wässrigen Lösung eines Amins, z.B. Mono-, Di- oder Triniederalkylamin, z.B. Aethyl-, Diäthyl- oder Triäthylamin, 2-Hydroxyäthyl-tri-$C_1$-$C_4$-alkylamin, z.B. Cholin, sowie einer basischen Aminosäure, z.B. Lysin oder Arginin, gebildet.

Ein Phospholipid der Formel 5 hat in erster Linie zwei Acyloxyreste $R_1$ und $R_2$, z.B. Alkanoyloxy oder Alkenoyloxy, z.B. Lauroyloxy, Myristoyloxy, Palmitoyloxy, Stearoyloxy, Arachinoyloxy, Oleoyloxy,

Linoyloxy oder Linoleoyloxy, und ist z.B. natürliches Lecithin ($R_3$ = Wasserstoff, $R_4$ = 2-Trimethylammoniumäthyl) oder Kephalin ($R_3$ = Wasserstoff, $R_4$ = 2-Ammoniumäthyl) mit verschiedenen Acyloxyresten $R_1$ und $R_2$, z.B. Ei-Lecithin oder -Kephalin oder Lecithin oder Kephalin aus Sojabohnen, synthetisches Lecithin oder Kephalin mit verschiedenen oder identischen Acyloxyresten $R_1$ und $R_2$, z.B. 1-Palmitoyl-2-oleoyl-lecithin oder -kephalin oder Dipalmitoyl-, Distearoyl-, Diarachinoyl-, Dioleoyl-, Dilinoyl- oder Dilinoleoyllecithin oder -kephalin, natürliches Phosphatidylserin ($R_3$ = Wasserstoff, $R_4$ = 2-Amino-2-carboxyäthyl) mit verschiedenen Acyloxyresten $R_1$ und $R_2$, z.B. Phosphatidylserin aus dem Rinderhirn, synthetisches Phosphatidylserin mit verschiedenen oder identischen Acyloxyresten $R_1$ und $R_2$, z.B. Dioleoyl-, Dimyristoyl- oder Dipalmitoylphosphatidylserin, oder natürliche Phosphatidsäure ($R_3$ und $R_4$ = Wasserstoff) mit verschiedenen Acyloxyresten $R_1$ und $R_2$.

Ein Phospholipid der Formel 5 ist ferner ein Phospholipid, worin $R_1$ und $R_2$ zwei identische Alkoxyreste, z.B. n-Tetradecyloxy oder n-Hexadecyloxy (synthetisches Ditetradecyl- oder Dihexadecyllecithin oder -kephalin), $R_1$ Alkenyl und $R_2$ Acyloxy, z.B. Myristoyloxy oder Palmitoyloxy (Plasmalogen, $R_3$ = Wasserstoff, $R_4$ = 2-Trimethylammoniumäthyl), $R_1$ Acyloxy und $R_2$ Hydroxy darstellen (natürliches oder synthetisches Lysolecithin oder Lysokephalin, z.B. 1-Myristoyl- oder 1-Palmitoyllysolecithin oder -kephalin, natürliches oder synthetisches Lysophosphatidylserin, $R_3$ = Wasserstoff, $R_4$ = 2-Amino-2-carboxyäthyl, z.B. Lysophosphatidylserin aus dem Rinderhirn oder 1-Myristoyl- oder 1-Palmitoyllysophosphatidylserin, synthetisches Lysophoshatidylglycerin, $R_3$ = Wasserstoff, $R_4$ = $CH_2OH-CHOH-CH_2-$, natürliche oder synthetische Lysophosphatidsäure, $R_3$ = Wasserstoff, $R_4$ = Wasserstoff, z.B. Ei-Lysophosphatidsäure oder 1-Lauroyl-, 1-Myristoyl- oder 1-Palmitoyllysophosphatidsäure).

Ein Lipid, welches einem Phospholipid analog ist, und statt des Phospholipids (II) mit der amphipatischen Verbindung (I) mit biologischer Wirkung homogen vermischt werden kann ist beispielsweise ein Lysolecithin-Analoges, z.B. 1-Lauroyl-1,3-propandiol-3-phos-

phorylcholin, ein Monoglycerid, z.B. Monoolein oder Monomyristin, ein Cerebrosid, ein Gangliosid oder ein Glycerid, welches keine freie oder veresterte Phosphoryl- oder Phosphonylgruppe in 3-Stellung enthält, z.B. ein Diacylglycerid oder 1-Alkenyl-1-hydroxy-2-acylglycerid mit den genannten Acyl- bzw. Alkenylgruppen, worin die 3-Hydroxygruppe durch einen der genannten Kohlenhydratreste, z.B. einen Galactosylrest, veräthert ist, z.B. Monogalactosylglycerin.

Zusammen mit der amphipathischen Verbindung (I) mit biologischer Wirkung, dem Phospholipid (II) oder dem analogen Lipid können zusätzlich neutrale Lipide homogen vermischt werden, welche in Zellmembranen enthalten und nur in unpolaren, organischen Lösungsmitteln, z.B. in Chloroform, löslich sind.

Solche neutralen Lipide sind z.B. Steroide, z.B. Oestradiol oder Sterine, z.B. Cholesterin, ß-Sitosterin, Desmosterin, 7-Ketocholesterin oder ß-Cholestanol, fettlösliche Vitamine, z.B. Vitamin $A_1$ und $A_2$, oder Vitamin E, $K_1$, $K_2$, $D_2$ oder $D_3$.

Die weiter vorn und im folgenden genannten Lipide mit einem chiralen C-Atom können sowohl als racemische Mischungen oder als optisch reine Enantiomere in den erfindungsgemäss herstellbaren pharmazeutischen Zusammensetzungen vorliegen.

Die Erfindung betrifft bevorzugt ein Verfahren zur Herstellung von pharmazeutischen Zusammensetzungen in Form einer wässrigen Dispersion enthaltend unilamellare Liposomen bestehend aus (I) Säureadditionssalzen von Antidepressiva der Formel

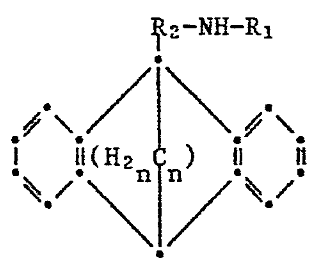

(1.8),

worin $R_1$ Niederalkyl, z.B. Methyl, $R_2$ Niederalkylen, z.B. Methylen, Aethylen oder 1,3-Propylen oder Hydroxyniederalkylen, z.B. 2-Hydroxy-1,3-propylen, und n null oder zwei bedeuten, Säureadditionssalzen von Antidepressiva der Formel

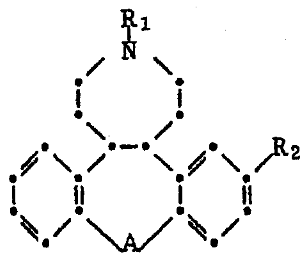

(1.9),

worin $R_1$ Niederalkyl, z.B. Methyl, A die Gruppe $N-R_1$, Sauerstoff oder Schwefel und $R_2$ Wasserstoff oder Cyan bedeuten, Säureadditionssalzen von Antidepressiva der Formel

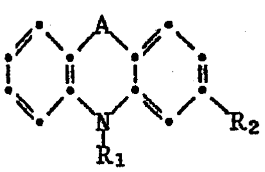

(1.10),

worin $R_1$ Niederalkylaminoniederalkyl, z.B. 3-Methylamino-n-propyl, Diniederalkylaminoniederalkyl, z.B. 3-Dimethylamino-n-propyl, oder 3-(4-(2-Hydroxyäthyl)-piperazin-1-yl)-n-propyl und A Aethylen oder Vinylen bedeutet, Säureadditionssalzen von Psychoanaleptica, Anoretica oder Adrenergica mit Phenylaminopropan- oder Cyclohexylaminopropanstruktur, z.B. Amphetamin, Methamphetamin, Benzphetamin, Propylhexedrin, Prolintan, Fencamfin, Methylphenidat, Pipradrol oder Phenmetrazin, Säureadditionssalzen von Spasmolytica wie Adiphenin, Säureadditionssalzen von Sympathomimetica der Formel 1.5, z.B. Epinephrin, Norepinephrin, Dopamin, Nordefrin, Ethylnorepinephrin, Isoprenalin, Isoethorin, Metaproterenol, Orciprenalin,Metaraminol, Phenylephrin, Hydroxyamphetamin, Methoxyphenamin, Ephedrin, Norephedrin, Pholedrin, Tyramin, Norfenefrin oder Octopamin, ß-Rezeptoren-Blockern der Formel 1.6, z.B. Acebutolol, Atenolol, Toliprolol, Alprenolol, Oxprenolol, Bunitrolol, Bupranolol, Talinolol, Phenbutolol, Bufetolol oder Varbian (R,S-Form und S-Form), Verbindungen mit Wirkung auf periphere Noradrenalinspeicher, z.B. Reserpin, Rescinnamin oder Syringopin, Glucocorticoiden, die in 21-Stellung mit einer Aminosäure verestert sind, z.B. Prednisolondiäthylaminoacetat, oder analgetisch wirksamen Phenylessigsäuresalzen, z.B. den Natriumsalzen von Dichlofenac und Pirprofen, und (II) einem Phospholipid der Formel 5 mit zwei Acyloxyresten $R_1$ und $R_2$, z.B. Lauroyloxy, Myristoyloxy, Palmitoyloxy, Stearoyloxy, Arachinoyloxy, Oleoyloxy, Linoyloxy oder Linoleoyloxy, z.B. natürlichem Lecithin ($R_3$ = Wasserstoff, $R_4$ = 2-Trimethylammoniumäthyl) oder natürlichem Kephalin ($R_3$ = Wasserstoff, $R_4$ = 2-Ammoniumäthyl) mit verschiedenen Acyloxyresten $R_1$ und $R_2$, z.B. Ei-Lecithin oder -Kephalin oder Lecithin oder Kephalin aus Sojabohnen, synthetischem Lecithin oder Kephalin mit verschiedenen oder identischen Acyloxyresten $R_1$ und $R_2$, z.B. 1-Palmitoyl-2-oleoyl-lecithin oder -kephalin oder Dipalmitoyl-, Distearoyl-, Diarachinoyl-, Dioleoyl-, Dilinoyl- oder Dilinoleoyllecithin oder -kephalin, natürlichem Phosphatidylserin ($R_3$ = Wasserstoff, $R_4$ = 2-Amino-2-carboxyäthyl) mit verschiedenen Acyloxyresten $R_1$ und $R_2$, z.B. Phosphatidylserin aus dem Rinderhirn, synthetischem Phosphatidylserin mit verschiedenen oder identischen Acyloxyresten $R_1$ und $R_2$, z.B. Dioleoyl-, Dimyristoyl- oder Dipalmitoylphosphatidylserin, oder natürlicher Phosphatidsäure ($R_3$ und $R_4$ = Wasserstoff) mit verschiedenen Acyloxyresten $R_1$ und $R_2$.

Die Erfindung betrifft in erster Linie ein Verfahren zur Herstellung von pharmazeutischen Zusammensetzungen in Form einer wässrigen Dispersion enthaltend unilamellare Liposomen bestehend aus (I) Säureadditionssalzen von Antidepressiva der Formel 1.8, z.B. 1-(2R-2-Hydroxy-3-methylaminopropyl)-dibenzo[b,e]bicyclo[2.2.2]-octadien, sowie das 2-R,S-Isomerengemisch, Maprotilin, Benzoctamin, Säureadditionssalzen von Antidepressiva der Formel 1.9, z.B. 3-Methyl-dibenz[2,3,:6,7]oxepino[4,5-d]azepinhydrochlorid, 7-Cyan-4-methyl-2,3,4,5-tetra-hydro-1H-dibenzo-[2,3:6,7]thiepino[4,5-d]azepin ethansulfonat, 3,10-Dimethyl-1,2,3,4,5,10-hexahydrodibenz[b,f]-azepino[4,5]azepin-maleat, Säureadditionssalzen von Antidepressiva der Formel 1.10, z.B. Clomipramin, Opipramol, Desipramin oder Imipramin bzw. Imipramin-N-oxid, Säureadditionssalzen von Sympathomimetica der Formel 1.5, z.B. Ephedrin oder Norephedrin, Säureadditionssalzen von ß-Rezeptoren-Blockern der Formel 1.6, z.B. 1-Isopropylamino-3-[4-(2-methylthioäthoxy)-phenoxy]-propan-2-ol, 1-Isopropylamino-3-(2-pyrrol-1-ylphenoxy)-propan-2-ol, Oxprenolol oder Prenalterol, Spasmolytica wie Adiphenin, Verbindungen mit Wirkung auf periphere Noradrenalinspeicher, z.B. Reserpin, Glucocorticoide, die in 21-Stellung mit einer Aminosäure verestert sind, z.B. Prednisolondiäthylaminoacetat, analgetisch wirksamen Phenylessigsäuresalzen, z.B. den Natriumsalzen von Diclofenac und Pirprofen, und (II) einem Phospholipid der Formel 5, z.B. natürlichem Lecithin oder Kephalin, synthetischem 1-Palmitoyl-2-oleoyl-lecithin oder -kephalin, Dipalmitoyl-, Distearoyl-, Diarachinoyl-, Dioleoyl-, Dilinoyl- oder Dilinoleoyllecithin oder -kephalin, natürlichem Phosphatidylserin, synthetischem 1-Palmitoyl-2-oeloyl, Phosphatidylserin, Dimyristoyl- oder Dipalmitoylphosphatidylserin oder natürlicher Phosphatidsäure.

Die Herstellung der homogenen Mischung der Komponenten kann in an sich bekannter Weise durch Film- oder Lyophilisatbildung erfolgen. Bei der Filmbildung löst man das Lipid, z.B. Sojalecithin, und die biologisch wirksame Verbindung, z.B. einen pharmazeutischen Wirkstoff, z.B. Diclofenac-Natriumsalz, in einem organischen Lösungsmittel auf. Durch Entfernen des organischen Lösungsmittels, am zweckmässigsten im Vakuum, vorzugsweise Hochvakuum, oder durch Abblasen mit Inertgas, z.B. Stickstoff, stellt man eine dünne Schicht der Komponenten her.

Die Auswahl der geeigneten Lösungsmittel zur Herstellung des Films ist von der Löslichkeit der Lipidkomponenten und der Einschlussverbindungen abhängig. Geeignete Lösungsmittel zur Herstellung der homogenen Mischung durch Filmbildung sind beispielsweise unsubstituierte oder substituierte, z.B. halogenierte, aliphatische, cycloaliphatische Kohlenwasserstoffe, z.B. n-Hexan, Cyclohexan, Methylenchlorid oder Chloroform, Alkohole, z.B. Methanol oder Aethanol, Niederalkancarbonsäureester, z.B. Essigsäureäthylester, oder Aether, z.B. Diäthyläther, oder Mischungen dieser Lösungsmittel. Man entfernt das Lösungsmittel im Vakuum, vorzugsweise Hochvakuum, oder durch Abblasen mit Inertgas, z.B. Stickstoff.

Die Lyophilisatbildung erfolgt durch Lyophiliseren einer Lösung des zu verkapselnden Wirkstoffs und der Lipidkomponeten auf die in der DE-A-2,818,655 beschriebene Weise. Geeignete Lösungsmittel liegen beim Gefriertrocknen, z.B. bei der Temperatur des Methanol-, Aethanol- oder Aceton-Trockeneis-Gemisches, zusammen mit den Lipidkomponenten und den Einschlussverbindungeen in festem Zustand vor und sind z.B. organische Lösungsmittel mit einem Schmelzpunkt höher als 0°C, z.B. Eisessig, Benzol oder Dioxan, insbesondere tert-Butanol.

Eine homogene Mischung lässt sich auch durch Sprühtrocknung einer Lösung eines kationischen Tensids, Phospholipids und einer Einschlussverbindung in einem organischen Lösungsmittel herstellen. Man erhält die homogene Mischung in Form eines Pulvers.

In der homogenen Mischung beträgt das ungefähre Molverhältnis biologisch wirksame Verbindung zu Lipid ca. 0,1 bis ca. 2 zu 1, bevorzugt ca. 0,8 bis ca. 1,2 zu 1.

Man dispergiert beispielsweise durch Schütteln (z.B. Vortex-Mischer) oder Rühren der wässrigen Phase, welche die zuvor hergestellte homogene Mischung enthält. Dabei findet die Bildung von unilamellaren Liposomen (KUL) und (GUL) spontan (spontaneous vesiculation), d.h. ohne zusätzliche Energiezufuhr von aussen und mit grosser Geschwindigkeit statt. Es können ca. 0,1-50 Gewichtsprozent, vorzugsweise ca. 2-20 Gewichtsprozent (bezogen auf das Gesamtgewicht der wässrigen Dispersion) der homogenen Mischung in wässriger Phase dispergiert werden.

Wässrige Dispersionen mit einem pH-Wert höher als ca. 8 werden nach dem Dispersionsvorgang neutralisiert, z.B. auf den physiologischen pH-Wert von 7,2. Die Neutralisation wird notwendig, um eine eventuelle Zerstörung des Wirkstoffs und/oder der Liposomen unter basischen Bedingungen zu vermeiden und um eine physiologische Verträglichkeit der applizierbaren wässrigen Dispersion mit der Liposomenmischung zu gewährleisten. Man neutralisiert z.B. mit einer physiologisch verträglichen, verdünnten wässrigen Lösung einer Säure oder einer Pufferlösung mit einem pH-Wert von 7 bis 8. Physiologisch verträgliche Säuren sind beispielsweise verdünnte wässrige Mineralsäuren, z.B. verdünnte Salzsäure, Schwefelsäure oder Phosphorsäure, oder verdünnte organische Säuren, z.B. Niederalkancarbonsäuren, z.B. Essigsäure.

Wässrige Dispersionen mit amphipatischen Verbindungen der Formel 1 können sauer reagieren. Diese neutralisiert man durch Zugabe von verdünnten, wässrigen Basen, z.B. verdünnter, wässriger Natrium-oder Kaliumhydroxid-Lösung oder einer Pufferlösung vom pH-Wert 7 bis 8, insbesondere pH 7,2.

Wässrige Dispersionen mit amphipatischen Verbindungen der Formeln 2 und 4 können basisch reagieren. Diese neutralisiert man durch Zugabe einer geeigneten, physiologisch annehmbaren Säure, z.B. einer schwachen organischen Säure, z.B. Essigsäure, oder einer verdünnten, wässrigen Mineralsäure, z.B. verdünnter, wässriger Schwefelsäure. Man neutralisiert unter gleichzeitiger Kontrolle des pH-Werts.

Man arbeitet zweckmässigerweise bei Raumtemperatur oder auch bei höheren Temperaturen, z.B. bis ca. 60°C, und unter Rühren oder Schütteln. Falls es die Empfindlichkeit des zu verkapselnden Wirkstoffs verlangt, führt man das Verfahren unter Kühlen und gegebenenfalls Inertgasatmosphäre, z.B. Stickstoff- oder Argonatmosphäre, durch.

Die Grösse der gebildeten unilamellaren Liposomen ist u.a. von der Struktur des Wirkstoffs und der Lipidkomponente, dem Mischungverhältnis der Komponenten und der Konzentration dieser Komponenten in der wässrigen Dispersion abhängig. So kann man beispielsweise durch Erhöhung oder Erniedrigung der Konzentration der Lipidkomponente wässrige Phasen mit einem hohen Anteil an kleinen oder grossen unilamellaren Liposomen herstellen. Zusätzlich zu KUL entstehen auch grosse unilamellare Liposomen (GUL-Durchmesser bis zu $5,0 \times 10^{-6}$ m) und gegebenenfalls multilamellare Liposomen.

Die Trennung der KUL von GUL und gegebenenfalls als Nebenprodukt gebildeter multilamellarer Liposomen, sofern erwünscht, erfolgt mittels herkömmlicher Trennmethoden, z.B. Sedimentation der GUL in der Ultrazentrifuge, Gelfiltration oder Extrusion durch geradporige Filter. Beispielsweise setzen sich Zentrifugieren z.B. 5-30 Minuten lang bei 5000-40000 xg in diesem Schwerefeld GUL ab, während die KUL dispergiert bleiben und dekantiert werden können. Nach mehrmaligem Zentrifugieren erreicht man eine vollständige Trennung der GUL von KUL.

Auch durch Gelfiltration z.B. mit Sepharose oder Sephacryl als Träger, kann man alle in der wässrigen Phase befindlichen Liposomen mit einem Durchmesser grösser als $6,0 \times 10^{-8}$ m, z.B. GUL oder multilamellare Liposomen, sowie nicht verkapselte Wirkstoffe und überschüssige, dispergierte Lipide, welche in hochmolekularen Aggregaten vorliegen, abtrennen und so eine wässrige Phase mit einer Fraktion KUL von relativ einheitlicher Grösse erhalten.

Durch Extrusion durch geradporige Filter, z.B. Membranfilter vom Typ Nucleopore® mit einem Porendurchmesser von ca. $5,0 \times 10^{-8}$ m, bei einem Druck von ca. 0,1 - 1,5 bar und einer Filtrationsgeschwindigkeit von ca. 20 ml/h, kann man eine besonders einheitliche Grössenverteilung der unilamellaren Liposomen erhalten. Das Filtrat kann anschliessend über ein Ultrafilter, z.B. Amicon UM 10®, mit unilamellaren Liposomen angereichert werden.

Die so erhältlichen Liposomen eignen sich für die Applikation am Patienten und sind in wässriger Phase relativ lange (bis zu mehreren Tagen oder Wochen) beständig. Wässrige Dispersionen mit den erfindungsgemäss herstellbaren unilamellaren Liposomen können nach Zusatz von Stabilisatoren, z.B. Mannit oder Lactose, lagerungsfähig gemacht werden.

Die erfolgte Bildung von kleinen unilamellaren Liposomen (KUL) und ihr Gehalt in wässriger Phase lässt sich in an sich bekannter Weise anhand verschiedener physikalischer Messmethoden nachweisen, z.B. mit gefriergebrochenen (freeze fracture) Proben und Dünnschnitten im Elektronenmikroskop oder durch Röntgendiffraktion, durch dynamische Lichtstreuung, durch Massenbestimmung des Filtrats in der analytischen Ultrazentrifuge und vor allem spektroskopisch, z.B. im Kernresonanzspektrum ($^{1}H$, $^{13}C$ und $^{31}P$). So ergeben beispielsweise scharfe Signale mit schmaler Linienbreite im Kernresonanzspektrum einen Hinweis auf erfolgte Bildung von unilamellaren Liposomen mit einem Durchmesser kleiner als ca. 1000 Å. Scharfe Signale bei $\delta$ ca. 0,89 ppm ($-CH_3$), $\delta$ ca. 1,28 ppm ($-CH_2-$) und $\delta$ ca. 3,23 ppm ($-N(CH_3)_3$) sind z.B. für verfahrensgemäss erhaltene kleine unilamellare Liposomen (Vesikel) mit Phosphatidylcholin (Lecithin) als Bestandteil charakteristisch. Im Kernresonanzspektrum sind solche Signale für unilamellare Liposomen typisch und unterscheiden sich deutlich von Signalen, welche von gemischten Mizellen, grossen unilamellaren und multilamellaren Liposomen verursacht werden. Grosse unilamellare und multilamellare Liposomen mit Lecithin als Komponente verursachen ein breites zusammenhängendes Methyl- und Methylensignal geringerer Intensität. Für gemischte Mizellen mit Lecithin als Komponente ist ein Methylsignal von $\delta$ ca. 0,89 ppm charakteristisch, welches zu einem Triplett aufgespalten ist und eine wesentlich geringere Linienbreite hat als das Methylsignal (Singlett, ebenfalls bei $\delta$ ca. 0,89 ppm), das von unilamellaren Liposomen stammt.

Wässrige Dispersionen mit den erfindungsgemäss erhältlichen Liposomen und verkapselten Wirkstoffen sind Verabreichungssysteme, welche sich, gegebenenfalls nach Konzentrierung oder Isolierung der Liposomen, z.B. durch Ultrazentrifugieren, zu therapeutischen Zwecken für die orale (p.o.), parenterale (i.v., i.m. oder i.p.) oder topikale Verabreichung eignen.

Bei oraler Verabreichung können Verabreichungssysteme auf Liposomenbasis die Resorption eines Wirkstoffs verbessern.

Für die orale Verabreichung kann die Liposomen-haltige wässrige Dispersion mit pharmazeutisch unbedenklichen Verdünnungsmitteln oder Trägern oder mit üblichen Zusätzen, z.B. Farb- oder Geschmackstoffen, vermischt oder als Sirup oder in Form von Kapseln angewendet werden.

Für die parenterale Verabreichung kann die wässrige Dispersion oder können die angereicherten Liposomen in einer geeigneten Trägerflüssigkeit, z.B. steriler, isotonischer Kochsalz- oder Glucoselösung, gegebenenfalls auf pH 7,2 abgepuffert, suspendiert sein.

Für die topikale Verabreichung wird die Liposomen-haltige wässrige Dispersion mit üblichen Verdickern, z.B. Hydroxypropylcellulose, geeigneten Konservierungsmitteln, Antioxydantien und Duftstoffen vermischt und als Lotion oder als Gel zur Applikation auf der Haut oder auf Schleimhäuten verwendet.

Die Dosismenge für den zu applizierenden Wirkstoff ist im Allgemeinen die für den betreffenden Wirkstoff für die jeweilige Applikationsform, das Alter des Patienten und den gesundheitlichen Zustand des Patienten z.B. im Deutschen Arzneimittelbuch (DAB), vorgeschriebene Höchst- und Mindestmenge. Wässrige Dispersionen mit erfindungsgemäss herstellbaren Liposomen haben aber auch den Vorteil, dass Wirkstoffe in geringeren Dosen appliziert zu den

Rezeptoren gelangen und dort einen therapeutischen Effekt bewirken können oder bei Applikation von höheren Dosen unerwünschte Nebenwirkungen vermieden werden können.

Die weiter vorn benannten Wirkstoffe sind bekannt. Wirkstoffe, deren Freinamen angegeben sind, sind kommerziell erhältlich. Die genannten Lipide, insbesondere die Phospholipide der Formel 5, sind bekannt und zum Teil kommerziell erhältlich.

Die folgenden Beispiele veranschaulichen die Erfindung ohne sie zu beschränken. Die Temperaturen sind in Celsiusgraden und chemische Verschiebungen im NMR-Spektrum in ppm vom Standard Tetramethylsilan angegeben. Die Signale sind, wenn nicht anders angegeben, Singuletts. Scharfe Singulett-Signale bei 1,26 - 1,32 ppm sind für die Methylengruppen des Phospholipids der Formel 5 charakteristisch, welches in wässriger Dispersion in Form von kleinen, unilamellaren Liposomen vorliegt. Phospholipide der Formel 5, welche in der wässrigen Dispersion in Form von grossen, unilamellaren oder multilamellaren Liposomen vorliegen, haben breite Signale von ca. 0,5 bis ca. 1,8 ppm. Aus dem Verhältnis der Intensitäten der Singulett-Signale der Methylengruppen des Lipids und dem Standard Natriumacetat lässt sich die Ausbeute an kleinen, unilamellaren Liposomen (KUL) berechnen. Für die Herstellung der wässrigen Dispersionen wird steriles, partikelfreies gewaschenes Wasser verwendet.

Beispiel 1:

1.1. 50 mg (ca. 0,066 mMol) Sojalecithin werden in eine 15 ml Viale eingewogen und mit einer Lösung von 20,33 mg (0,066 mMol) 1-Isopropylamino-3-(2-pyrrol-1-ylphenoxy)-propan-2-ol-hydrochlorid in 3 ml einer Methanol-Chloroform-1:1-Mischung versetzt. Nach Lösen des Lecithins in der organischen Phase wird die Viale in horizontaler Lage so schnell in Rotation versetzt, bis auf der Glaswand ein Flüssigkeitsfilm haftenbleibt. Durch Abblasen mit Stickstoff wird das Lösungsmittel entfernt und der gebildete Lipidfilm mehrere Stunden im Hochvakuum getrocknet.

1.2. Dieser Lipidfilm wird anschliessend mit 1,5 ml Wasser oder $D_2O$ (wenn die Aufnahme eines $^1H$-NMR-Spektrums beabsichtigt ist) versetzt und die Viale mehrere Minuten lang, gegebenenfalls maschinell und hochtourig (Vortex-Mischer) geschüttelt. Man erhält eine leicht opaleszierende, wässrige Dispersion.

Die erfolgte Bildung von kleinen, unilamellaren Liposomen (KUL) ist im $^1H$-NMR-Spektrum (360 MHz) u.a. durch ein scharfes Singulett-Signal bei 0,53 ppm, welches für die Methylgruppen des Lipids Sojalecithin charakteristisch ist, zu erkennen. Das Spektrum zeigt ausserdem im Bereich von 0,53 bis 1,63 ppm ein breites Signal mit geringerer Intensität, welches den Methyl- und Methylengruppen des in grossen unilamellaren und multilamellaren Liposomen enthaltenen Sojalecithins zugeordnet wird. Neben weiteren Signalen ist das Dublett bei 1,26, welches der $-CH(CH_3)_2$-Gruppe des Wirkstoffs zugeordnet wird, charakteristisch. Ausbeute an KUL: 23,4 %.

Die gebildeten unilamellaren Liposomen können im Elektronenmikroskop sichtbar gemacht werden. Die Liposomendispersion wird zunächst der üblichen Gefierbruchmethode (freeze-fracture) unterzogen. Es liegen hauptsächlich zwei "Populationen" von unilamellaren Liposomen vor, die sich durch ihre durchschnittliche Grösse unterscheiden:

1. Kleine unilamellare Liposomen (KUL) mit einem Durchmesser von ca. $2,0-6,0 \times 10^{-8}$ m und

2. Grosse unilamellare Liposomen (GUL) mit einem Durchmesser von ca. $1,0 \times 10^{-7} - 1,0 \times 10^{-6}$ m.

Beispiel 2:

Analog Beispiel 1.1 stellt man einen Lipidfilm aus 50 mg (ca. 0,066 mMol) Sojalecithin und einer äquimolaren Menge der folgenden Wirkstoffe her und dispergiert diesen analog Beispiel 1.2 in 2,5 ml Wasser bzw. $D_2O$. Die $^1H$-NMR-Spektren zeigen scharfe Singuletts für die Methyl- und Methylengruppen des in Form von KUL

vorliegenden Lecithins und ein breites Signal geringerer Intensität für die Methyl- und Methylengruppen des in Form von GUL und multilamellaren Liposomen vorliegenden Lecithins. Ausserdem sind die für den jeweiligen Wirkstoff charakteristischen Signale erkennbar.

2.1. 21,15 mg 1-(2R-2-Hydroxy-3-methylaminopropyl)-dibenzo[b,e]-bicyclo[2.2.2]octadien-hydrochlorid (NMR: 3,30 $-NHCH_3$), Ausbeute KUL: 12,4 %.

2.2. 21,15 mg 1-(2R,S-2-Hydroxy-3-methylaminopropyl)-dibenzo[b,e]-bicyclo[2.2.2]octadien-hydochlorid (NMR: 3,26 $-NHCH_3$), Ausbeute KUL: 22,0 %.

2.3. 20,06 mg 3-Methyl-dibenz[2,3:6,7]oxepino[4,5-d]azepin-hydrochlorid (NMR: 3,19 $-NCH_3$), Ausbeute KUL: 26 %.

2.4. 25,48 mg 2,3,4,5-Tetrahydro-3-methyl-1H-dibenzo[2,3:6,7]-thiepino[4,5-d]azepin-7-cyanmethansulfonat (NMR: 3,14 $-NCH_3$), Ausbeute KUL: 29,5 %.

2.5. 26,80 mg 3,10-Dimethyl-1,2,3,4,5,10-hexahydro-dibenz[b,f]-azepino[4,5]azepin-maleat (NMR: 1,38 $-CH(\underline{CH_3})_2$-Dublett, 2,19 $-SCH_3$), Ausbeute KUL: 25 %.

2.6. 46,99 mg 1-Isopropylamino-3-[4-(2-methylthioäthoxy)-phenoxy]-propan-2-ol-fumarat (NMR: 1,38 $-CH(\underline{CH_3})_2$ -Dublett, 2,19 $-SCH_3$), Ausbeute KUL: 25 %.

2.7. 20,262 mg N,N-Dimethyl-5-phenyl-1,2,4-triazolo[1,5-a]-chinoxalin-2-ylmethylamin-hydrochlorid (NMR: 3,04 $-NCH_3$, 2,91 $-N(CH_3)_2$), Ausbeute KUL: 21,2 %.

2.8. 21,12 mg Clomipraminhydrochlorid (NMR: 3,20 $-N(CH_3)_2$), Ausbeute KUL: 29 %.

2.9. 13,21 mg Ephedrinhydrochlorid (NMR: 2,77 $-NHCH_3$), Ausbeute KUL: 18 %.

2.10. 26,33 mg Opipramol (NMR: 7,10 -CH=CH- Multiplett), Ausbeute KUL: 26 %.

2.11. 20,65 mg Maprotilinhydrochlorid (NMR: 3,17 $-NHCH_3$), Ausbeute KUL: 15,1 %.

2.12. 12,38 mg Phenylpropanolaminhydrochlorid (Norephedrinhydrochlorid, NMR: 1,19 $-NCH_3$), Ausbeute KUL: 10,8 %.

2.13. 42,81 mg Oxprenololsuccinat (NMR: 1,30 $-CH_3$-Dublett), Ausbeute KUL: 24 %.

2.14. 19,92 mg Desipraminhydrochlorid (NMR: 6,77 - 7,44, Multiplettarom. H), Ausbeute KUL: 17,7 %.

2.15. 20,91 mg Phentolaminhydrochlorid (NMR: 2,30 $-C_6H_5-\underline{CH_3}$), Ausbeute KUL: 12,2 %.

2.16. 18,80 mg Benzobutaminhydrochlorid (NMR: 3,67 $-NCH_3$), Ausbeute KUL: 10,2 %.

2.17. 20,84 mg Imipraminhydrochlorid (NMR: 3,07 $-NCH_3$), Ausbeute KUL: 24,1 %.

2.18. 22,96 mg Adipheninhydrochlorid (NMR: 2,00 $-COOCH_2-$), Ausbeute KUL: 56 %.

2.19. 30,08 mg Oxprenololhydrochlorid (NMR: 1,33 $-CH_3$ -Dublett), Ausbeute KUL: 16 %.

2.20. 17,19 mg Prenalterolhydrochlorid (NMR: 1,33 $-CH_3$ -Dublett, 6,87 arom. H-Multiplett), Ausbeute KUL: 16 %.

2.21. 18,67 mg Diclofenac-Natrium (NMR: 3,00 $-CH_2-$), Ausbeute KUL: 16 %.

2.22. 17,74 mg Methylphenidathydrochlorid (NMR: 3,73 $-COOCH_3$), Ausbeute KUL: 10,3 %.

Beispiel 3:

3.1. 23,26 mg (ca. 0,066 mMol) Cefroxadin werden in 5 ml einer Dioxan-Methanol-2:1-Mischung in Gegenwart geringer Spuren Wasser gelöst. In der klaren Lösung werden 50 mg (ca. 0,066 mMol) Sojalecithin gelöst. Das Lösungsmittel wird im Vakuum bei 50° abgezogen. Man trocknet den gebildeten Lipidfilm mehrere Stunden im Hochvakuum.

3.2. Dieser Film wird anschliessend mit 2,5 ml Wasser oder $D_2O$ (wenn die Aufnahme eines $^1H$-NMR-Spektrums beabsichtigt ist) versetzt und mehrere Minuten lang, gegebenenfalls maschinell und hochtourig, geschüttelt. Nach Zugabe eines Tropfens einer 1 %igen Lösung von Bromthymolblau in $D_2O$ wird mit wenigen Tropfen einer 1 N NaOH- oder NaOD $-D_2O$-Lösung auf den Farbumschlag von gelb nach blau titriert (pH ca. 9-10). Nach mehrere Minuten langem, maschinellem Rühren wird die Dispersion mit 1 N $H_2SO_4$ oder $D_2SO_4$-$D_2O$-Lösung neutralisiert (Farbumschlag von blau nach gelb). Man erhält eine leicht opaleszierende, wässrige Dispersion. Im NMR Spektrum ist neben den Methyl- und Methylensignalen, welche für das Lipid charakteristisch sind, und weiteren Signalen bei 3,73 ppm ein Singulett zu erkennen, welches der $OCH_3$-Gruppe des Cefroxadins zugeordnet wird. Ausbeute KUL: 13,2 %.

Beispiel 4:

4.1. 50 mg (ca. 0,066 mMol) Sojalecithin werden in eine 15 ml Viale eingewogen und mit einer Lösung von 16,53 mg (0,066 mMol) Pirprofen in 3 ml eines Methanol-Chloroform-1:1-Gemisches versetzt. Nach Lösen des Lecithins in der organischen Phase wird die Viale so schnell in Rotation versetzt, bis auf der Glaswand ein Flüssigkeitsfilm

haftenbleibt. Durch Abblasen mit Stickstoff wird das Lösungsmittel entfernt und der gebildete Lipidfilm mehrere Stunden im Hochvakuum getrocknet.

4.2. Dieser Film wird anschliessend mit 2,5 ml Wasser oder $D_2O$ (wenn die Aufnahme eines $^1H$-NMR-Spektrums beabsichtigt ist) versetzt und mehrere Minuten lang, gegebenenfalls maschinell, geschüttelt. Nach Zugabe eines Tropfens Bromthymolblau in $D_2O$ wird mit wenigen Tropfen einer 1 N NaOD-$D_2O$-Lösung auf den Farbumschlag von gelb nach blau titriert (pH ca. 9-10). Nach mehrere Minuten langem, maschinellem Rühren wird die Dispersion mit 1 N $H_2SO_4$ oder $D_2SO_4$-$D_2O$-Lösung neutralisiert (Farbumschlag von blau nach gelb). Man erhält eine leicht opaleszierende, wässrige Dispersion. Im NMR-Spektrum ist neben den Methyl-und Methylensignalen, welche für das Phospholipid charakteristisch sind, neben weiteren Signalen ein breites Singulett bei 6,04 ppm zu erkennen, welches den Vinylprotonen im Pirprofen zugeordnet wird. Ausbeute KUL: ca. 11,6 %.

Beispiel 5:

5.1. Analog Beispiel 4.1 wird ein Flüssigkeitsfilm aus 50 mg (ca. 0,066 mMol) Sojalecithin und 40,17 mg (0,066 mMol) Reserpin hergestellt, welchen man anschliessend im Hochvakuum trocknet.

5.2. Dieser Film wird anschliessend mit 25 ml Wasser oder $D_2O$ (wenn die Aufnahme eines $^1H$-NMR-Spektrums beabsichtigt ist) versetzt und mehrere Minuten lang, gegebenenfalls maschinell und hochtourig, geschüttelt. Nach Zugabe eines Tropfens einer 0,5 %igen Lösung von Bromphenolblau in $D_2O$ wird mit wenigen Tropfen einer 1 N $H_2SO_4$ oder $D_2SO_4$-$D_2O$-Lösung auf den Farbumschlag von blau nach gelb titriert (pH ca. 3). Nach mehrere Minuten langem, maschinellem Rühren wird die Dispersion mit 1 N NaOH oder NaOD-$D_2O$-Lösung neutralisiert. Man erhält eine leicht opaleszierende, wässrige Phase. Im NMR-Spektrum ist neben den für das Lipid charakteristischen Methyl- und Methylensignalen und weiteren Signalen bei 3,81 ppm ein Singulet zu erkennen, welches den $-OCH_3$-Gruppen am Phenylring des Reserpins zugeordnet wird.

Beispiel 6:

6.1. Analog Beispiel 4.1 wird ein Flüssigkeitsfilm aus 50 mg (ca. 0,066 mMol) Sojalecithin und 31,26 mg (ca. 0,066 mMol Prednisolondiäthylaminoacetat hergestellt, welchen man anschliessend im Hochvakuum trocknet.

6.2. Aus diesem Film wird anschliessend analog Beispiel 5.1 eine wässrige Dispersion mit unilamellaren Liposomen hergestellt. Im NMR-Spektrum ist neben den für das Lipid charakteristischen Methyl- und Methylensignalen und weiteren Signalen bei 7,66 ppm ein Singulett zu erkennen, welches dem vinylischen, zur Carbonylgruppe α-ständigen Proton des Prednisolongerüsts zugeordnet wird.

Beispiel 7:

7.1. 2,9 g (3,88 mMol) Sojalecithin werden in 20 ml tert-Butanol bei ca. 50° gelöst. Bei dieser Temperatur werden unter Rühren 2 ml Wasser zugetropft und anschliessend 2 g (3,88 mMol) Betamethasondinatriumphosphat in der Lösung gelöst. Die klare Lösung wird bei -30° eingefroren und bei dieser Temperatur gefriergetrocknet.

7.2. Das erhältliche Lyophilisat wird mit 2,5 ml Wasser versetzt und 5 Minuten mechanisch geschüttelt. Man erhält eine leicht opaleszierende, wässrige Dispersion mit unilamellaren Liposomen. Im $^1H$-NMR-Spektrum sind das Methylen- und das Methylsignal des Lipids, sowie unter anderem ein Dublett bei 7,49 (J = 9 Hz, 1-H-Atom am Steroidgerüst), Dublett bei 6,38 (J = 9 Hz 2-H-Atom) und ein Singulett bei 6,19 (4-H-Atom) zu erkennen. Ausbeute KUL: 44 %.

7.3. Die erhältliche wässrige Dispersion wird durch Zugabe von steriler 0,1 N Salzsäure auf pH 7.4 gebracht. Nach Einfüllen in eine gerührte Ultrafiltrationszelle (Amicon®), die anstelle des Ultrafilters mit einem geradporigen Membranfilter aus Polycarbonat (Nucleopore®) mit einem Porendurchmesser von 0.05 µm versehen ist und partikelfrei gewaschen wurde, wird unter geringem Ueberdruck von ca. 0,1 - 1,5 bar und stetiger Zufuhr von sterilfiltrierter Pufferlösung nach Dulbecco (pH 7,4 ohne Ca und Mg) mit einer Geschwindigkeit von 20 ml/h filtriert, bis man ca. 500 ml Filtrat erhält. Dieses Filtrat wird in eine gerührte Filterzelle, die mit einem Ultrafilter, z.B. Amicon U 10®, bestückt ist, kontinuierlich eingespeist und auf ein Volumen von 30 ml konzentriert. Die konzentrierte wässrige Dispersion enthält kleine, unilamellare Liposomen und kann nach Zugabe eines Konzentrates von Phosphatpuffer nach Dulbecco (pH 7,4 ohne Ca und Mg) ampulliert und für Behandlungsversuche eingesetzt werden.

7.4. Analog Beispiel 7.3 lassen sich durch Membranfiltration und anschliessende Ultrafiltration konzentrierte wässrige Dispersionen enthaltend kleine unilamellare Liposomen mit den in Beispiel 1-6 und 8 angegebenen Zusammensetzungen herstellen.

Beispiel 8:

8.1. Analog Beispiel 7.1 stellt man ein Lyophilisat aus 3,21 g (4,22 mMol) Sojalecithin und 2 g (4,22 mMol) Prednisolondiäthylaminoacetat her.

8.2. Analog Beispiel 7.2 dispergiert man das erhältliche Lyophilisat in Wasser und erhält eine leicht opaleszierende, wässrige Dispersion mit unilamellaren Liposomen. Im $^{1}H$-NMR-Spektrum sind das Methyl- und das Methylensignal des Lipids zu erkennen, sowie unter anderem ein breites Signal bei 6,31 (2-H-Atom am Steroidgerüst) und ein Singulett bei 6,06 (4-H-Atom). Ausbeute KUL: 14,4 %.

Beispiel 9 (Antiinflammatorisches Steroid-Injektionspräparat):
29 g 1-Palmitoyl-2-oleoyl-lecithin werden in 450 ml tert-Butanol bei 50° gelöst. Zu dieser Lösung gibt man unter Rühren 20 ml destilliertes Wasser und 20 g Betamethasondinatriumphosphat. Die erhaltene Lösung wird im Sterilraum durch ein Sterilfilter (z.B. Acrodisc 0,2 μm) in eine sterile, partikelfrei gewaschene Flasche mit Pipettendosieraufsatz filtriert. Je 0,1 ml dieser Lösung (entsprechend 4 mg Wirkstoff) werden in gewaschene, sterile 2 ml Vialen gefüllt, steril lyophilisiert und unter trockenem Stickstoff verschlossen. Das erhältliche Trockenpräparat ist lagerstabil. Vor dem Gebrauch wird zu diesem Trockenpräparat mit einer sterilen Spritze 1 ml sterile, phosphatgepufferte (pH 7,4) Kochsalzlösung gegeben und die Viale auf einem standardisierten Laborschüttler (Vortex, Stufe 6) 1 Minute geschüttelt. Die entstandene Liposomendispersion eignet sich zur intramuskulären, intraartikulären, intradermalen und intraläsionalen Injektion.

Beispiel 10 (Steroid-Crème):
29 g Sojalecithin (Epikuron 200®) werden in 200 ml tert-Butanol bei 50° gelöst. Bei dieser Temperatur werden unter Rühren 20 ml dest. Wasser und 20 mg Betamethasondinatriumphosphat hinzugefügt. Die Lösung wird gefroren, lyophilisiert und kalt unter Stickstoffatmosphäre (trocken) gemahlen. Das Lyophilisat wird im Rührgefäss innerhalb 10 Minuten in 5 kg dest. Wasser gerührt. Im Moltomat wird aus 14,4 kg Wasser, 300 g Klucel, 200 g Natriumascorbat, sowie üblichen Parfümen und Konservierungsmittelzusätzen in an sich bekannter Weise ein wässriges Gel hergestellt und die Lipsomenmasse zugemischt. Die erhaltene Crème eignet sich zur Behandlung nässender Dermatosen.

Beispiel 11 (Antirheumaticum für perorale Applikation):
1 kg Sojalecithin (Epikuron 200®) werden in 5 l tert-Butanol bei 50° gelöst. Bei dieser Temperatur werden 250 ml Wasser und 250 g Diclofenac-Natrium hinzugefügt. Die Lösung wird lyophilisiert und das Lyophilisat unter Stickstoffatmosphäre (trocken) in der Stiftmühle fein gemahlen und im Turbula-Mischer mit 2,5 kg gemahlener

Lactose, 5 g Natriumascorbat und Geschmackskorrigentien vermischt. Je 750 mg der Pulvermischung werden in Verbundfolienbeutel (Polyäthylen/Aluminium/Papier) eingesiegelt. Vor Einnahme wird der Inhalt des Beutels in einem Glas Wasser (1 dl) aufgerührt, wobei eine Liposomendispersion zum Trinken entsteht.

Beispiel 12: (Augentropfen gegen Bindehautentzündungen):
20 g 1-Palmitoyl-2-oleoyl-lecithin werden in 400 ml tert-Butanol gelöst. Man fügt 40 ml dest. Wasser und 2 g Diclofenac-Natrium hinzu. Die Lösung wird im Sterilraum durch ein Sterilfilter (z.B. Acrodisc 0,2 µm) in eine sterile, partikelfrei gewaschene Flasche mit Pipettenaufsatz filtriert. 1 ml Portionen dieser Lösung (entsprechend 50 mg Phospholipid) werden in gewaschene, sterile 50 ml Vialen gefüllt, bei -70° gefroren, steril lyophilisiert und unter trockenem Stickstoff verschlossen. Das so erhältliche Trockenpräparat ist lagerstabil. Vor dem Gebrauch werden zum Trockenpräparat 2,5 ml sterile, phosphatgepufferte Kochsalzlösung (nach Dulbecco, Ca und Mg-frei, pH 7,4) gegeben und die Viale ca. 20 Sekunden kräftig geschüttelt. Die erhältliche Suspension ist verschlossen 1 Monat im Kühlschrank haltbar. Zur Behandlung werden täglich 1-2 Tropfen auf jedes Auge appliziert.

Ansprüche

1. Verfahren zur Herstellung von pharmazeutischen Zusammensetzungen in Form einer wässrigen Dispersion enthaltend unilamellare Liposomen bestehend aus (I) einer amphipatischen Verbindung mit biologischer Wirkung und (II) einem Phospholipid oder einem analogen Lipid und gegebenenfalls einem zusätzlichen Lipid, dadurch gekennzeichnet, dass man (I) die amphipatische Verbindung mit biologischer Wirkung und (II) das Phospholipid oder das analoge Lipid und gegebenenfalls das zusätzliche Lipid homogen mischt und die erhältliche homogene Mischung in wässriger Phase dispergiert und, wenn notwendig, die erhältliche wässriger Dispersion neutralisiert und, wenn erwünscht, die erhältlichen unilamellaren Lipsomen anreichert und/oder abtrennt.

2. Verfahren gemäss Anspruch 1 dadurch gekennzeichnet, dass man (I) als amphipatische Verbindung mit biologischer Wirkung eine substituierte Ammoniumverbindung der Formel

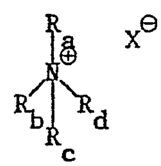

(1),

worin a) $R_a$ eine hydrophobe Gruppe und $R_b$, $R_c$ und $R_d$ unabhängig voneinander Wasserstoff, $C_1$-$C_4$-Alkyl, 2-Hydroxyäthyl, Allyl oder Cyclo-$C_3$-$C_6$-alkyl-$C_1$-$C_3$-alkyl oder zwei der Reste $R_b$, $R_c$ und $R_d$ zusammen gegebenenfalls durch -HN-, -N($C_1$-$C_4$-Alkyl)-, -N(2-Hydroxyäthyl)- oder Sauerstoff unterbrochenes $C_4$- oder $C_5$-Alkylen oder

b) $R_a$ und $R_b$ zwei hydrophobe Gruppen oder zusammen eine hydrophobe Gruppe und $R_c$ und $R_d$ unabhängig voneinander Wasserstoff, $C_1$-$C_4$-Alkyl, Allyl oder Cyclo-$C_3$-$C_6$-alkyl-$C_1$-$C_3$-alkyl oder

c) $R_a$, $R_b$ und $R_c$ zusammen eine hydrophobe Gruppe und $R_d$ Wasserstoff oder $C_1$-$C_4$-Alkyl und X⊖ das Anion einer pharmazeutisch annehmbaren Säure bedeutet,

ein Carbonsäuresalz der Formel

$$R_a\text{-}COO^{\ominus}\ Y^{\oplus} \qquad (2),$$

worin $R_a$ eine hydrophobe Gruppe darstellt und $Y^+$ das Kation einer pharmazeutisch annehmbaren Base ist,

eine α-Aminosäure-Verbindung der Formel

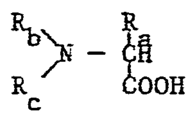

(3),

worin $R_a$ eine hydrophobe Gruppe und $R_b$ und $R_c$ unabhängig voneinander Wasserstoff oder $C_1$-$C_4$-Alkyl darstellen,

ein Phosphorsäuremonoester der Formel

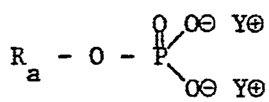

(4),

worin $R_a$ eine hydrophobe Gruppe und Y⊕ das Kation einer pharmazeutisch annehmbaren Base darstellt, oder

Säureadditionssalz einer Verbindung mit einer hydrophoben Gruppe $R_a$ und einer Imidazolin-, Imidazolidin- oder Hydrazinogruppe als hydrophiler Gruppe und (II) als Phospholiquid eine Verbindung der Formel

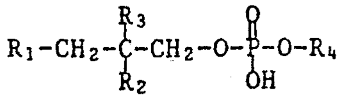 (5),

worin einer der Reste $R_1$ und $R_2$ Wasserstoff, Hydroxy, $C_1$-$C_4$-Alkyl und der andere Rest Alkyl, Alkenyl, Alkoxy, Alkenyloxy oder Acyloxy mit je 10 bis 20 C-Atomen oder beide Reste $R_1$ und $R_2$ Alkyl, Alkenyl, Alkoxy, Alkenyloxy oder Acyloxy mit je 10 bis 20 C-Atomen, $R_3$ Wasserstoff oder $C_1$-$C_4$-Alkyl, und $R_4$ Wasserstoff, gegebenenfalls substituiertes $C_1$-$C_7$-Alkyl oder einen Kohlenhydratrest mit 5 bis 12 C-Atomen oder, wenn beide Reste $R_1$ und $R_2$ Wasserstoff oder Hydroxy bedeuten, einen Steroidrest bedeutet, oder ein Salz davon homogen mischt und dispergiert.

3. Verfahren gemäss Anspruch 2, dadurch gekennzeichnet, dass man (I) eine substituierte Ammoniumverbindung der Formel 1, worin a) die hydrophobe Gruppe ein aliphatischer Kohlenwasserstoffrest sein kann, der durch ein Sauerstoff- oder Schwefelatom unterbrochen, die Gruppen -CO(=O)-, -O-C(=O)-, -C(=O)-NH-, -O-C(=O)-NH- oder Hydroxy enthalten und durch 1-3 gegebenenfalls substituierte, monocyclische, aliphatische oder aromatische Kohlenwasserstoffreste, einen gegebenenfalls substitierten, bi- oder tricyclischen, aromatischen oder partiell gesättigten Kohlenwasserstoffrest, einen gegebenenfalls substituierten, monocyclischen, aromatischen, partiell gesättigten oder gesättigten Heterocyclus oder einen gegebenenfalls substituierten, bi- oder tricyclischen, aromatischen, partiell gesättigten oder Benzo-kondensierten Heterocyclus substituiert sein kann, oder ein gegebenenfalls substituierter, monocyclischer, aliphatischer oder aromatischer oder ein bicyclischer, aliphatischer oder Benzo-kondensierter Kohlenwasserstoffrest sein kann und die hydrophile Gruppe beispielsweise eine Gruppe der Formel

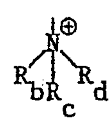

ist, worin $R_b$, $R_c$ und $R_d$ unabhängig voneinander Wasserstoff, $C_1$-$C_4$-Alkyl, z.B. Methyl, Aethyl, Isopropyl oder n-Propyl, oder 2-Hydroxyäthyl oder worin zwei der Reste $R_b$, $R_c$ und $R_d$ zusammen Piperidino, Piperazinyl, 1-Methylpiperazinyl, 1-(2-Hydroxy äthyl)-piperazinyl oder Morpholino und der andere Rest Wasserstoff bedeutet, sein kann,

b) die hydrophoben Gruppen, $R_a$ und $R_b$ zwei aliphatische Kohlenwasserstoffreste sein können, welche durch einen oder zwei gegebenenfalls substituierte, monocyclische, aliphatische oder aromatische Kohlenwasserstoffreste oder durch einen gegebenenfalls substituierten, monocyclischen, aromatischen, partiell gesättigten oder gesättigten Heterocyclus substituiert sein können, oder $R_a$ und $R_b$ zusammen einen gegebenenfalls substituierten, monocyclischen, aromatischen, gesättigten, partiell gesättigten oder Benzo-kondensierten Heterocyclus darstellen und die hydrophile Gruppe eine Gruppe der Formel

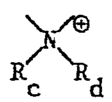

ist, worin $R_c$ und $R_d$ unabhängig voneinander Wasserstoff oder $C_1$-$C_4$-Alkyl, bedeuten, oder

c) die hydrophobe Gruppe zusammen $R_a$, $R_b$ und $R_c$ gebildet wird und einen gegebenenfalls substituierten, aromatischen, partiell gesättigten oder Benzo-kondensierten Heterocyclus darstellt und die hydrophile Gruppe eine Gruppe der Formel

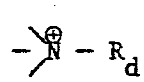

ist, worin $R_d$ Wasserstoff oder $C_1$-$C_4$-Alkyl, vorzugsweise Methyl, bedeutet, und $X^{\ominus}$ das Anion einer pharmazeutisch annehmbaren Säure ist, oder ein Carbonsäuresalz der Formel 2, worin die hydrophobe Gruppe $R_a$ ein aliphatischer Kohlenwasserstoffrest sein kann, der durch einen gegebenenfalls substituierten, monocyclischen, aromatischen Kohlenwasserstoffrest oder einen gegebenenfalls substituierten, bi- oder tricyclischen, aromatischen oder partiell gesättigten Kohlenwaserstoffrest, einen gegebenenfalls substituierten, monocyclischen, aromatischen oder partiell gesättigten Heterocyclus oder einen gegebenenfalls substituierten, bi- oder tricyclischen, aromatischen, partiell gesättigten oder Benzo-kondensierten Heterocyclus oder einen Steroidrest substituiert sein kann, oder ein gegebenenfalls substituierter, monocyclischer, aromatischer Kohlenwasserstoffrest, ein gegebenenfalls substituierter, bi- oder tricyclischer, aromatischer oder partiell gesättigter Kohlenwasserstoffrest, ein gegebenenfalls substituierter, monocyclischer, aromatischer oder partiell gesättigter Heterocyclus oder ein gegebenenfalls substituierter, bi- oder tricyclischer, aromatischer, partiell gesättigter oder Benzo-kondensierter Heterocyclus, sein kann und $Y^{\oplus}$ das Kation einer pharmazeutisch annehmbaren Base ist, und (II) als Phospholipid eine Verbindung der Formel 5,worin beide Reste $R_1$ und $R_2$ Alkyl, Alkenyl, Alkoxy, Alkenyloxy oder Acyloxy mit je 10 bis 20 C-Atomen, $R_3$ Wasserstoff oder $C_1$-$C_4$-Alkyl, und $R_4$ Wasserstoff, gegebenenfalls substituiertes $C_1$-$C_7$-Alkyl oder einen Kohlehydratrest mit 5 bis 12 C-Atomen bedeutet, oder ein Salz davon homogen mischt und dispergiert.

4. Verfahren gemäss einem der Ansprüche 1 bis 4, dadurch gekennzeichnet, dass man (I) als substituierte Ammoniumverbindung oder als entsprechende darin durch Salzbildung überführbare Aminoverbindung eine Verbindung aus der Gruppe der Parasympathomimetica mit quaternären oder tertiären Aminogruppen, Cholinesterasehemmer mit zwei tertiären Aminogruppen oder mit einer quaternären Ammoniumgruppe, Neutrotransmitter mit einer quaternären Ammoniumgruppe, Serotin-Antagonisten, worin die hydrophile Gruppe eine primäre oder tertiäre

Aminogruppe ist und die hydrophobe Gruppe eine Indol-3-yläthyl-Struktur hat, Analgetica vom Morphin-Typ mit tertiärer Aminogruppe und deren Antagonisten der Formel

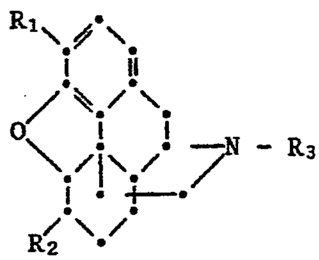

(1.1),

worin $R_1$, $R_2$ und $R_3$ folgende Bedeutungen haben:

| $R_1$ | $R_2$ | $R_3$ | Name |
|---|---|---|---|
| -OH | -OH | $-CH_3$ | Morphin |
| -OH | =O | $-CH_3$ | Hydromorphon |
| -OH | =O | $-CH_3$ | Oxymorphon |
| -OH | -H | $-CH_3$ | Levorphanol |
| $-OCH_3$ | -OH | $-CH_3$ | Codein |
| $-OCH_3$ | =O | $-CH_3$ | Hydrocodon |
| $-OCH_3$ | =O | $-CH_3$ | Oxycodon |
| -OH | -OH | Allyl | Nalorphin |
| -OH | =O | Allyl | Naloxon |
| -OH | =O | Cyclopropylmethyl | Naltrexon |
| -OH | $-OCH_3$ | Cyclopropylmethyl | Buprenophin |
| -OH | -H | Cyclobutylmethyl | Butorphanol |
| -OH | -OH | Cyclobutylmethyl | Nalbuphin |
| -2-(Morpholin-1-yl)-äthyl) | -OH | $-CH_3$ | Pholcodin, |

Analgetica von Benzomorphan-Typ mit tertiärer Aminogruppe der Formel

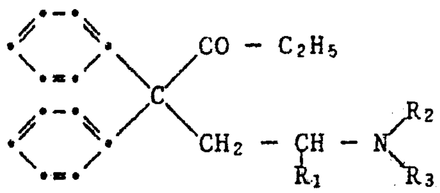

(1.2),

worin $R_1$ Wasserstoff oder Methyl, $R_2$ und $R_3$ Methyl oder $R_2$ und $R_3$ zusammen Morpholino oder Piperidino darstellen, oder Analoga davon mit Pseudomethadon-Struktur, Morphin-ähnliche Analgetica mit einer aliphatischen oder cycloaliphatischen tertiären Aminogruppe, Analgetica vom Benzimidazol-Typ, z.B. der Formel

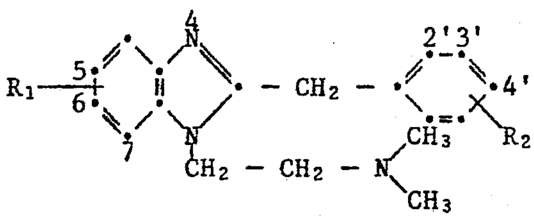

(1.3),

worin $R_1$ 5-, 6- oder 7-Nitro, $R_2$ Wasserstoff, 3'- oder 4'-Methoxy, 4'-Aethoxy, 4'-Isopropoxy, 4'-Methyl oder 4'-Chlor bedeuten, Lokalanästhetica, worin $R_a$ und $R_b$ der Formel 1 zusammen mit dem Stickstoff einen Piperidylrest bilden, der durch eine Niederalkylenbrücke, z.B. 1,3-Propylen, substituiert ist, welche durch eine Methoxycarbonyl- und Benzoyloxy-Gruppe substituiert ist, Lokalanästhetica, worin die hydrophobe Gruppe $R_a$ der Formel (1) die 4-Aminobenzoyloxyäthyl-, 4-Amino-2-chlor-, 2-n-Butoxy- oder 2-Hydroxybenzoyloxyäthyl-, 4-Amino-3-n-butoxybenzoyloxyäthyl-, 3-Amino-4-n-butoxybenzoyloxyäthyl-, 2-Aminobenzoyloxyäthyl-, 2-(4-Aminobenzoyloxy)-6-methyl-n-pentyl-, 4-Aminobenzoyloxy-n-propyl-, 4-n-Butylaminobenzoyloxyäthyl-, 4-n-Butyl-2-hydroxybenzoyloxyäthyl-, 3-(4-n-Propoxybenzoyloxy-2-hydroxy)-propyl-, 2-n-Benzoyloxy-n-propyl-, 2-(2-Acetoxybenzoyloxy)-n-propyl-, Benzoyloxy-n-propyl-, 4-Cyclohexyloxybenzoyloxyäthyl-, 4-Aethyl- oder 4-n-Butylbenzoyloxyäthyl-, 2-n-Butoxychinol-4-ylcarbonyloxyäthyl-, 2,4-Dimethylanilinocarbonylmethyl-, 2-Aethyl-, 2-Chlor- oder 2-Methoxycarbonylmethyl-4-methylanilinocarbonylmethyl-, 1-(2-Methylanilinocarbonyl)-äthyl-, (2-Aethoxycarbonyl-4-methylthien-3-ylaminocarbonyl)-äthyl-, 2,3-Dianilinocarbonyloxypropyl-, 4-n-Propyl- oder 4-n-Butylbenzoyläthyl-, 4-Phenoxymethylphenyl-n-butyl-, 4-n-Butoxyphenoxy-n-propyl-, 2-n-Butylchinol-8-yloxymethyl- oder die 8-Benzoyloxycarbonyl-1,2,3,4-tetrahydronaphth-2-yl-Gruppe ist und die hydrophile Gruppe Niederalkylamino, z.B. Methyl-, Aethyl-, Isopropyl-, oder n-Butylamino, Diniederalkylamino, z.B. Dimethyl-, Diäthyl- oder Di-n-propylamino, Cyclohexylamino, 1-Methylpiperid-2-yl, Piperid-1-oder -2-yl oder Morpholin-1-yl ist, Neuroleptica und/oder Thymoleptica, worin die unpolare, hydrophobe Gruppe $R_a$ der Formel 1 Niederalkyl oder Hydroxyniederalkyl ist, das durch 2-Cyan-, 2-Methoxy-, 2-Chlor-, 2-Trifluormethyl-, 2-Methylthio-, 2-Acetyl- oder 2-Aethyl-10H-phenothiazin-10-yl, 9H-Acridin-10-yl, 5H-Dibenzo[b,f]azepin-5-yl, 7-Chlor-10,11-dihydro-5H-dibenzo[b,f]-zepin-5-yl, 5,10-Dihydro-5-methyl-11-dibenzo[b,e]-1,4-diazepin-11-onyl, 2-Chlor-, 2-Trifluormethyl-oder 2-Dimethylaminosulfonyl-9H-thioxanthen-9-yliden, 10,11-Dihydro-5H-dibenzo[a,d]cyclohepten-5-yl oder 10H-Pyrido[3,2-b]-[1,4]benzothiazin-10-yl substituiert ist und die polare, hydrophile Gruppe Amino, Niederalkylamino, Diniederalkylamino, Triniederalkylamino, Piperidino oder 4-Hydroxyäthylpiperazino bedeutet, Antidepressiva mit tertiärer Aminogruppe Thymeretica mit einer primären oder durch Methyl und Propargyl substituierten tertiären Aminogruppe, Sedativa, worin die hydrophobe Gruppe $R_a$ der Formel 1 der 2-(7-Chlor-5-o-fluorphenyl-1,3-dihydro-2H-1,4-benzodiazepin-2-on-1-yl)-äthyl-Rest und die hydrophile Gruppe Diäthylamino ist, z.B. Flurazepam, Psychodysleptica mit ß-Phenylethylamin-Struktur, Psychodysleptica worin die hydrophobe Gruppe $R_a$ der Formel 1 ein durch einen 3-Indolrest substituierter Aethylrest ist, Psychodysleptica, worin $R_a$ und $R_b$ der Formel 1 zusammen mit dem Stickstoff einen Morpholin oder Pyrrolidinring bilden, der durch 1,3-Niederalkylen substituiert ist, Anticholinergica mit Atropinstruktur, Anticholinergica (Parkinsonmittel) der Formel

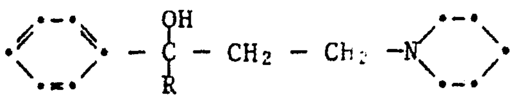 (1.4),

worin R Cyclohexyl, Cyclopentyl, Phenyl oder Norborn-5-en-2-yl bedeutet, sowie Analoga wie Procyclidin, Anticholinergica mit einer tertiären Aminogruppe, zentrale Analeptica mit einer Morpholingruppe, z.B. Psychoanaleptica mit Phenylaminopropanstruktur, . Psychoanaleptica mit einer 4-Chlorphenoxyacetoxyäthylgruppe als hydrophober und einer Dimethylaminogruppe als hydrophiler Gruppe, Vasodilatatoren mit einer tertiären Aminogruppe, Appetitzügler mit Amphetamin-Struktur, Muskelrelaxantien mit einer hydrophoben und mehreren quartären Aminogruppen, Neurotrope Spasmolytica mit quartären Aminogruppen, muskulotrope Spasmolytica mit tertiären Aminogruppen, 4-Aminochinolin-Antirheumatica, Antioestrogene mit tertiärer Aminogruppe, Histamin-$H_1$-Receptor-Antagonisten (Antihistaminica) mit einer Aethylendiamingruppe, einer 2-Aminoäthanol-Gruppe, oder einer 3-Aminopropan-Gruppe, Sympathomimetica der Formel

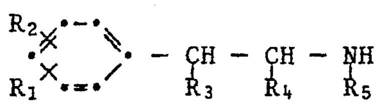 (1.5),

worin $R_1$, $R_2$, $R_3$, $R_4$ und $R_5$ folgende Bedeutungen haben:

| $R_1$ | $R_2$ | $R_3$ | $R_4$ | $R_5$ | Name |
|---|---|---|---|---|---|
| 3-OH | 4-OH | -OH | -H | $-CH_3$ | Epinephrin (Adrenalin) |
| 3-OH | 4-OH | -OH | -H | -H | Norepinephrin (Noradrenalin) |
| 3-OH | 4-OH | -H | -H | -H | Dopamin |
| 3-OH | 4-OH | -OH | $-CH_3$ | -H | Nordefrin |

| $R_1$ | $R_2$ | $R_3$ | $R_4$ | $R_5$ | Name |
|---|---|---|---|---|---|
| 3-OH | 4-OH | -OH | $-C_2H_5$ | -H | Ethylnorepinephrin |
| 3-OH | 4-OH | -OH | -H | $-CH(CH_3)_2$ | Isoprenalin |
| 3-OH | 4-OH | -OH | $-C_2H_5$ | $-CH(CH_3)_2$ | Isoethorin |
| 3-OH | 4-OH | -OH | -H | $-CH(CH_3)_2$ | Metaproterenol |
| 3-OH | 5-OH | -OH | -H | $-C(CH_3)_3$ | Orciprenalin |
| 3-OH | -H | -OH | $-CH_3$ | -H | Metaraminol |
| 3-OH | -H | -OH | -H | $-CH_3$ | Phenylephrin |
| 4-OH | -H | -H | -H | -H | Hydroxyamphetamin |
| $2-OCH_3$ | -H | -H | $-CH_3$ | $-CH_3$ | Methoxyphenamin |
| $2-OCH_3$ | $5-OCH_3$ | -OH | $-CH_3$ | -H | Methoxamin |
| $3-CH_2OH$ | 4-OH | -OH | -H | $-C(CH_3)_3$ | Albuterol |
| -H | -H | -OH | $-CH_3$ | $-CH_3$ | Ephedrin |
| -H | -H | -OH | $-CH_3$ | -H | Norephedrin |
| $3-CF_3$ | -H | -H | $-CH_3$ | $-C_2H_5$ | Fenfluramin |
| -H | -H | -OH | $-CH_3$ | -H | Phenylpropanolamin |
| 4-OH | -H | -OH | $-CH_3$ | $CH_3$ | Pholedrin |
| 4-OH | -H | -OH | $-CH_3$ | -H | Tyramin |
| 3-Cl | 4-Cl | -OH | -H | $-C(CH_3)_3$ | Dichlorisoprenalin |
| 4-OH | -H | -OH | -H | $-CH_3$ | Norfenefrin |
| 4-OH | -H | -OH | -H | H | Octopamin |
| 3-OH | -H | -OH | -H | $-C_2H_5$ | Etilefrin, |

ß-Receptoren-Blocker der Formel:

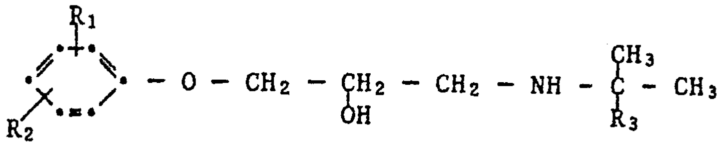 (1.6),

worin $R_1$, $R_2$ und $R_3$ folgende Bedeutungen haben:

| $R_1$ | $R_2$ | $R_3$ | Name |
|---|---|---|---|
| 2-Acetyl | 4-n-Butyrylamino | H | Acebutolol |
| 4-Carbamoylmethyl | H | H | Atenolol |
| 4-(2-Carbamoyläthyl) | H | H | Metoprolol |
| 3-Methyl | H | H | Toliprolol |
| 2-Allyl | H | H | Alprenolol |
| 2-Allyloxy | H | H | Oxprenolol |
| 2-Cyan | H | Methyl | Bunitrolol |
| 2-Chlor | 5-Methyl | Methyl | Bupranolol |
| 3-(N-cyclohexyl-N'-ureido) | H | Methyl | Talinolol |
| 2-Cyclopentyl | H | Methyl | Phenbutolol |
| 2-Tetrahydrofur-2-ylmethoxy | H | Methyl | Bufetolol |
| 2-Pyrrol-1-yl | H | H | |
| 4-(2-Methylthio-äthoxy) | H | H | |
| 4-OH | H | H | Varbian, R,S-Form, S-Form, |

ß-Blocker mit dem Naphthyloxy-, Indolyloxy-, 2-Methylindolyloxy-, 1,2,3,4-Tetrahydronaphth-2,3-diol-1-yl- oder 1,2,3,4-Tetrahydronaphth-5-on-1-yl-Rest als hydrophobe Gruppe, ß-Blocker, worin das Segment

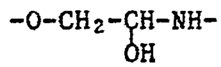

durch

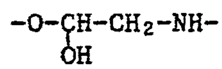

ersetzt ist, Verbindungen mit Wirkung auf periphere Noradrenalinspeicher, z.B. Verbindungen vom Reserpin-Typ, Tetracyclin-Antibiotica der Formel:

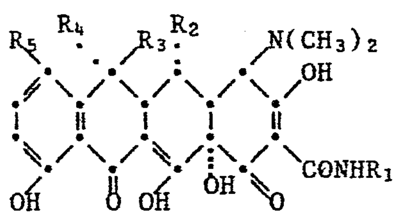

(1.7),

worin $R_1$ Wasserstoff oder Pyrrolidin-1-ylmethyl, $R_2$ Wasserstoff oder Hydroxy, $R_3$ Wasserstoff, Hydroxy oder Methyl, $R_4$ Wasserstoff oder Methyl und $R_5$ Wasserstoff, Chlor oder Dimethylamino bedeuten, Antimalariamittel vom Chinin-Typ, sowie Analoga mit 8-Aminochinolin-, 4-Aminochinolin-, 9-Aminoacridin-, 1,3,5-Triazin- oder Pyrimidin-Struktur, Antischistosomatica, worin die hydrophobe, unpolare Gruppe gegebenenfalls 6-Chlor- und/oder 4-Methyl- oder 4-Hydroxymethyl substituiertes Xanthonyl oder Thioxanthonyl ist und die hydrophile, polare Gruppe Diäthylamino ist, antivirale Mittel des Typs cyclische Amine, sowie Glucocorticoide, die in 21-Stellung mit einer Aminosäure verestert sind, oder als Carbonsäuresalze der Formel 2 mit biologischer Wirkung oder darin durch Salzbildung überführbare Carbonsäuren Salze von Glucocorticoiden, die in 21-Stellung mit einer Dicarbonsäure verestert sind, Kurznarcotica vom 3,20-Dioxo-5ß-pregnan-Typ, welche durch Bernsteinsäure verestert

sein können, Salze von Choleritica, analgetisch wirksame Salze von substituierten Phenylessigsäuren oder 2-Phenylpropionsäuren, analgetisch wirksame Anthranilsäure-Derivate, z.B. der Formel

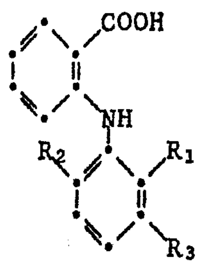

(2.1),

worin $R_1$, $R_2$ und $R_3$ unabhängig voneinander Wasserstoff, Methyl, Chlor oder Trifluormethyl bedeuten, analgetisch wirksame Anilino-substituierte Nicotinsäure-Derivate, analgetisch wirksame Heteroarylessigsäuren oder 2-Heteroarylpropionsäuren mit einem 2-Indol-3-yl- oder Pyrrol-2-yl-Rest, analgetisch wirksame Indenylessigsäuren, analgetisch wirksame Heteroaryloxyessigsäuren, Prostansäuren, welche die glatte Muskulatur stimulieren, Penicillansäure- und Cephalosporansäurederivate mit antibiotischer Wirkung mit 6ß- bzw. 7ß-Acylaminogruppen, welche in fermentativ, halb- oder totalsynthetisch erhältlichen 6ß-Acylaminopenicillansäure- oder 7ß-Acylaminocephalosporansäurederivaten oder in 3-Stellung abgewandelten 7ß-Acylaminocephalosporansäure derivaten vorhanden sind, sowie andere ß-Lactam-Antibiotica, Antineoplastica mit 4-[Bis-(2-chlorethyl)-aminophenyl]-butter säure-Struktur, oder Antineoplastica mit zwei Carboxygruppen, oder als Verbindungen der Formel 3 Neurotransmitter, worin die hydrophobe Gruppe durch Hydroxyphenyl substituiertes Methyl ist, Schilddrüsenhormone mit Jod-substituierten Phenylresten, oder Antineoplastica mit Aminosäurestruktur, oder als Verbindung der Formel 4 Betamethasondinatriumphosphat, Dexamethasondinatriumphosphat, Cortisonphosphat, Hydrocortisonphosphat, Prednisolon-di-natrium phosphat oder Paramethason-21-dinatriumphosphat, oder als salzartige Verbindungen mit einer hydrophoben Gruppe und einer hydrophilen Imidazolin-, Imidazolidin- oder Hydrazinogruppe Salze von antidepressiv wirksamen Hydrazinderivaten, z.B. α-Sympathomimetica mit

Imidazolin-Struktur, α-Sympatholytica mit Imidazolin-Struktur, zentral wirkende Antihypertensiva mit Imidazolin-Struktur, Vasodilatatoren mit einer Hydrazino-Gruppe, und (II) als Phospholipid eine Verbindung der Formel

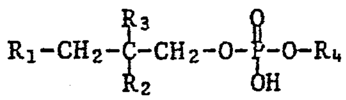

(5),

worin beide Reste $R_1$ und $R_2$ Alkyl, Alkenyl, Alkoxy, Alkenyloxy oder Acyloxy mit je 10 bis 20 C-Atomen, $R_3$ Wasserstoff oder $C_1$-$C_4$-Alkyl, und $R_4$ Wasserstoff, gegebenenfalls substituiertes $C_1$-$C_7$-Alkyl oder einen Kohlehydratrest mit 5 bis 12 C-Atomen bedeuten, oder ein Salz davon homogen mischt und dispergiert.

5. Verfahren gemäss Anspruch 4, dadurch gekennzeichnet, dass man (I) als substituierte Ammoniumverbindung oder als entsprechende darin durch Salzbildung überführbare Aminoverbindung Acetylcholinchlorid, Methacholinchlorid, Carbachol, Muscarin, Pilocarpin, Arecolin, Physostigmin, Neostigmin, Pyridostigminbromid, Serotonin, Histamin, Tryptamin, Bufotenin, Psilocybin, Morphin, Hydromorphon, Oxymorphon, Levorphanol, Codein, Hydrocodon, Oxycodon, Nalorphin, Naloxon, Naltrexon, Buprenophin, Butorphanol, Nalbiphin, Pholcodin, Pentazocin, Ketamin, Metazocin, Pentazocin, Cyclazocin, Pethidin, Cetobemidon, Alphaphrodin, Ethoheptazin, Prodilidin, Profadol, Methadon, Normethadon, Isomethadon, Dipipanon, Phenadoxon, Dimepheptanol, Dextromoramid, D-Propoxyphen, 1-Benzyl-2-dimethylaminomethyl-1-propanoyloxytetralin, Tramadol, Dimethylthiambuten, Diampromid, Phenampromid, Propiram, Tilidin, Metopholin, Etonitazen, Ergotamin, Dihydroergotamin, Dihydroergocryptin, Methysergid, Lisurid, Dimetiotazin, Dizotifen, Oxetoron, Cyproheptadin, Procain, Chlorprocain,Hydroxyprocain, Propoxycain, Oxybuprocain, Propoxymetacain, Piridocain, Leucinocain, Butacain, Tetracain, Hydroxytetracain, Cornecain, Edan, Piperocain, Cyclomethycain, Parethoxycain, Stadacain, Cinchocain, Lidocain, Pyrrocain, Granocain, Butanilicain, Tolycain, Mepivacain, Bupivacain,

Prilocain, Carticain, Dipiperidon, Propicocain, Dyclonin, Pramocain, Fomocain, Quinisocain, Profenamin, Promethazin, Periciazin, Perimethazin, Chlorpromazin, Perphenazin, Prochlorperazin, Triflumpromazin, Trifluoperazin, Fluphenazin, Thioridazin, Mesoridazin, Piperacetazin, Acetophenazin, Ethymemazin, Dimetacrin, Opipramol, Chlomipramin, Imipramin, Desimipramin, Trimipramin, Chlorprotixen, Thiotixen, Amitriptylin, Nortriptylin, Doxepin, Thiepin, Protriptylin, Protripendyl, Femoxetin, Citalopram, Zimelidin, Trebenzomin, Viloxazin, Nomifensin, Femoxetin, Tranylcypromin, Pargylin, Etryptamin, Flurazepam, Mescalin, $N_{\alpha}$,$N_{\alpha}$-Dimethyltryptamin, Bufotenin, Psilocin, Psilocylein, Scopolamin, Atropin, Benzatropin, Trihexyphenidyl, Cycrimin, Pridinol, Biperidin, Procyclidin, Caramiphen, Phenglutarimid, Orphenadrin, Chlorphenoxamin, Metixen, Doxapram, Amphetamin, Methamphetamin, Propylhexedrin, Prolintan, Fencamfamin, Methylphenidat, Pipradrol, Phenmetrazin, Diäthylpropion, Meclofenoxat, Naftidrofuryl, Dexamphetamin, Phentermin, Chlorphentermin, Fenfluramin, Amfepramon, Phenmetrazin, Phendimetrazin, Tubocumarin, Alcuroniumchlorid, Gallamintriethjodid, Hexcarbacholinbromid, Pancuroniumbromid, Suxamethoniumchlorid, Decamethoniumbromid, Scopolaminbutylbromid, Bevoniummethylsulfat, Valethamatbromid, Methantelinbromid, Camylofin, Hexahydroadiphenin, Adiphenin, Fencarbamid, Benzydamin, Ditaxol, Chloroquin, Tamoxifen, Ethamoxytriphetol, Phenbenzamin, Tripelenamin, Chlorpyramin, Mepyramin, Metaphenilen, Metapyrilen, Chloropyrilen, Histpyrroclin, Bamipin, Thenalidin, Clemizol, Methdilaazin, Isothipendyl, Oxomenazin, Diphenhydramin, Medrylamin, Chlorphenoxamin, Silachlorphenoxamin, Carbinoxamin, Diphenpyralin, Clemastin, Amethobenzepin, Pheniramin, Chlorphenamin, Brompheniramin, Triprolidin, Cycliramin, Phenindamin, Dimetinden, Cyproheptadin, Ketotifen, Epinephrin (Adrenalin), Norepinephrin (Noradrenalin) Dopamin, Nordefrin, Ethylnorepinephrin, Isoprenalin, Isoetharin, Metaproterenol, Orciprenalin, Metaraminol, Phenylephrin, Hydroxyamphetamin, Methoxyphenamin, Methoxamin, Albuterol, Ephedrin, Norephedrin, Fenfluramin, Phenylpropanolamin, Pholedrin, Tyramin, Dichlorisoprenalin, Norfenefrin, Octopamin, Etilefrin, Acebutolol, Atenolol, Metoprolol, Toliprolol, Alprenolol, Oxprenolol, Bunitrolol,

Bupranolol, Talinolol, Phenbutolol, Bufetolol, Varbian (R,S- oder S-Form) Propanolol, Indenolol,Pindolol, Mepindolol, Nadolol, Bunolol, Sofalol, Nifenalol, Cabetalol, Bufenalol, Reserpin, Rescinnamin, Syringopin, Chlortetracyclin, Oxytetracyclin, Tetracyclin, Demethylchlortetracyclin, Metacyclin, Doxycyclin, Minocyclin, Rolitetracyclin, Chinin, Conchinidin, Chinidin, Cinchonin, Pamaquin, Primaquin, Pentaquin, Chloroquin, Santoquin, Hydroxychloroquin, Amodiaquin, Mepacrin, Biguanid-1,3,5-Triazin, Proguanil, Bromguanil, Chloroproguanil, Nitroguanil, Cycloguanilembonat, Pyrimethamin, Trimethoprim, Lucanthon, Hycanthon, Miracil A oder B, Amantadin,Cyclooctylamin, Rimantadin, Predinisolon-diäthylaminoacetat, und (II) als Phospholipid der Formel 5 natürliches Lecithin ($R_3$ = Wasserstoff und $R_4$ = 2-Trimethylammoniumäthyl, natürliches Kephalin ($R_3$ = Wasserstoff, $R_4$ = 2-Ammoniumäthyl) mit verschiedenen Acyloxyresten $R_1$ und $R_2$ synthetisches Lecithin oder Kephalin mit verschiedenen oder identischen Acyloxyresten $R_1$ und $R_2$ natürliches Phsophatidylserin ($R_3$ = Wasserstoff, $R_4$ = -2-Amino-2-carboxyäthyl) mit verschiedenen Acyloxyresten $R_1$ und $R_2$, synthetisches Phosphatidylserin mit verschiedenen oder identischen Acyloxyresten $R_1$ und $R_2$, oder natürliche Phosphatidsäure ($R_3$ und $R_4$ = Wasserstoff mit verschiedenen Acyloxyresten $R_1$ und $R_2$ homogen mischt und dispergiert.

6. Verfahren gemäss Anspruch 4, dadurch gekennzeichnet, dass man als substituierte Ammoniumverbindung oder als entsprechende darin durch Salzbildung überführbare Aminoverbindung eine Verbindung aus der Gruppe der Säureadditionssalze von Antidepressiva der Formel

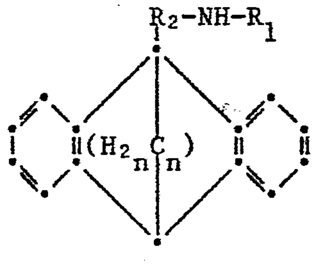

(1.8),

worin $R_1$ Niederalkyl, z.B. Methyl, $R_2$ Niederalkylen, Hydroxyniederalkylen und n null oder zwei bedeutet, Säureadditionssalze von Antidepressiva der Formel

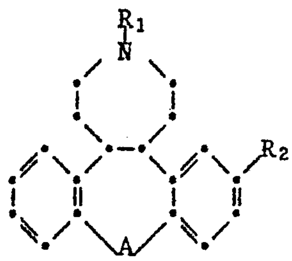

(1.9),

worin $R_1$ Niederalkyl, A die Gruppe 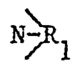, Sauerstoff oder Schwefel und $R_2$ Wasserstoff oder Cyan bedeutet, Säureadditionssalze von Antidepressiva der Formel

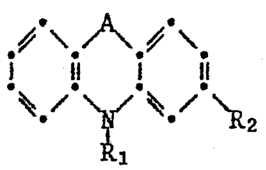

(1.10),

worin $R_1$ Niederalkylaminoniederalkyl, Diniederalkylaminoniederalkyl oder 3-(4-(2-Hydroxyäthyl)-piperazin-1-yl)-n-propyl und A Aethylen oder Vinylen bedeutet, oder Säureadditionssalze von Amphetamin, Methamphetamin, Benzphetamin, Propylhexedrin, Prolintan, Fencamfin, Methylphenidat, Pipradrol, Phenmetrazin, Adiphenin, Epinephrin, Norepinephrin, Dopamin, Nordefrin, Ethylnorepinephrin, Isoprenalin, Isoethorin, Metaproterenol, Orciprenalin,Metaraminol, Phenylephrin, Hydroxyamphetamin, Methoxyphenamin, Ephedrin, Norephedrin, Pholedrin, Tyramin, Norfenefrin, Octopamin, Acebutolol, Atenolol, Toliprolol, Alprenolol, Oxprenolol, Bunitrolol, Bupranolol, Talinolol, Phenbutolol, Bufetolol, Varbian (R,S-Form und S-Form), Reserpin, Rescinnamin, Syringopin oder Prednisolondiäthylaminoacetat und (II) als Phospholipid der Formel 5 natürliches Lecithin oder Kephalin, synthetisches 1-Palmitoyl-2-oleoyllecithin oder -kephalin, Dipalmitoyl-, Diostearoyl-, Diarachinoyl-, Dioleoyl-, Dilinoyl- oder Dilinoleyllecithin oder -kephalin, natürliches

Phosphatidylserin, synthetisches 1-Palmitoyl-2-oleoylphosphatidylserin, Dimyristoyl- oder Dipalmitoylphosphatidylserin oder natürliche Phophatidsäure homogen mischt und dispergiert.

7. Verfahren gemäss Anspruch 4, dadurch gekennzeichnet, dass man als substituierte Ammonium-Verbindung der Formel 1 oder als entsprechende darin durch Salzbildung überführbare Aminoverbindung 1-(2R-2-Hydroxy-3-methylaminopropyl)dibenzo b,e]bicyclo[2.2.2]octadien, sowie das 2 R,S-Isomerengemisch, Maprotilin, Benzoctamin, 3-Methyldibenz[2,3:6,7]oxepino[4,5-d]azepin-hydro hlorid, 7-Cyan-3-methyl-2,3,4,5-tetrahydro-1H-dibenzo 2,3:6,7]thiepino[4,5-d]azepinmethansulfonat, 3,10-Dimethyl-1,2,3,4,5,10-hexa hydrodibenz[b,f]azepino 4,5]azepin-maleat, Clomipramin, Opipramol, Desipramin, Imipramin bzw. Imipramin-N-oxid, Ephedrin, Norephedrin, 1-Isopropylamino-3-[4-(2-methylthioäthoxy)-phenoxy]-propan-2-ol, 1-Isopropylamino-3-(2-pyrrol-1-ylphenoxy)-propan-2-ol, Oxprenolol, Prenalterol, Adiphenin, Prednisolondiäthylaminoacetat oder Reserpin und (II) als Phospholiquid der Formel 5 natürliches Lecithin der Kephalin, synthetisches 1-Palmitoyl-2-oleoyllecithin oder -kephalin, Dipalmitoyl-, Distearoyl-, Diarachinoyl-, Dioleoyl-, Dilinoyl- oder Dilinoleyllecithin oder -kephalin, natürliches Phosphatidylserin synthetisches 1-Palmitoyl-2-oleoylphosphatidylserin, Dimyristoyl- oder Dipalmitoylphosphatidylserin oder natürliche Phophatidsäure homogen mischt und dispergiert.

8. Verfahren gemäss Anspruch 4, dadurch gekennzeichnet, dass man als Carbonsäuresalz oder darin durch Salzbildung überführbare Carbonsäure-Verbindung Methylprednisolon-natriumsuccinat, Prednisolonnatriumsuccinat, 3,20-Dioxo-5ß-pregnan, Hydroxydionsuccinat-Natrium, 11,20-Dioxo-3α-hydroxy-5α-pregnan, Alphadolon, ein Cholsäure- oder Desoxycholsäuresalz, Alclofenac, Ibufenac, Ibuprofen, Clindanac, Fenclorac, Ketoprofen, Fenoprofen, Indoprofen, Fenclofenac, Diclofenac, Flurbiprofen, Pirprofen, Naproxan, Benoxaprofen, Carprofen, Cicloprofen, Mefenaminsäure, Flufenaminsäure, Tolfenaminsäure, Meclofenaminsäure, Milflumin säure, Clonixin, Flunixin, Indometacin, Oxmetacin, Intrazol, Acemetazin, Cinmetacin, Zomepirac,

 0152379

Tolmetin, Colpirac, Tiaprofensäure, Benzadac, $PGE_2$ (Dinoproston), $PGF_{2}\alpha$ (Dinoprost), 15 (S)-15-Methyl-$PGE_2$, 15 (S)-15-Methyl-$PGF_{2}\alpha$ (Carboprost), (±)15 (Xi)-15-Methyl-13,14-dihydro-11-desoxy-$PGE_1$ (Deprostil), 15 (S)-15-Methyl-11-desoxy-$PGE_1$ (Doxaprost), 16,16-Dimethyl-$PGE_2$, 17-Phenyl-18,19,20-trinor-$PGF_{2}\alpha$, 16-Phenoxy-17,18,19,-20-tetranor-$PGF_{2}\alpha$ oder N-Methylsulfonyl-16-phenoxy-17,18,19,20-tetranor-$PGF_{2}\alpha$ (Sulproston), Nalixidinsäure, Cinoxacin, Oxolinsäure, Pironidsäure, Pipenidsäure, Penicillin G oder V, Phenethicillin, Propicillin, Nafcillin, Oxacillin, Cloxazillin, Dicloxacillin, Flucloxacillin, Cyclazillin, Epicillin, Mecillinam, Methicillin, Azlocillin, Sulbenicillin, Ticarcillin, Mezlocillin, Piperacillin, Carindacillin, Azidocillin, Ciclazillin, Cefaclor, Cefuroxim, Cefazlur, Cephacetril, Cefazolin, Cephalexin, Cefadroxil, Cephaloglycin, Cefoxitin,Cephaloridin, Cefsulodin, Cefotiam, Ceftazidin, Cefonicid, Cefotaxim, Cefmenoxim, Ceftizoxim, Cephalothin, Cephradin, Cefamandol, Cephanon, Cephapirin, Cefroxadin, Cefatrizin, Cefazedon, Ceftrixon, Ceforanid, Moxalactam, Clavulansäure, Nocardicin A, Sulbactam, Aztreonam, Thienamycin, Chlorambucil oder Methotrexat und (II) als Phospholipid der Formel 5 natürliches Lecithin der Kephalin, synthetisches 1-Palmitoyl-2-oleoyllecithin oder -kephalin, Dipalmitoyl-, Distearoyl-, Diarachinoyl-, Dioleoyl-, Dilinoyl- oder Dilinoleyllecithin oder -kephalin, natürliches Phosphatidylserin, synthetisches 1-Palmitoyl-2-oleoylphosphatidylserin, Dimyristoyl- oder Dipalmitoylphosphatidylserin oder natürliche Phophatidsäure homogen mischt und dispergiert.

9. Verfahren gemäss Anspruch 8, dadurch gekennzeichnet, dass man als Carbonsäuresalz oder darin durch Salzbildung überführbare Carbonsäuren die Natriumsalze von Diclofenac und Pirprofen und (II) als Phospholipid der Formel 5 natürliches Lecithin der Kephalin, synthetisches 1-Palmitoyl-2-oleoyllecithin oder -kephalin, Dipalmitoyl-, Distearoyl-, Diarachinoyl-, Dioleoyl-, Dilinoyl- oder Dilinoleyllecithin oder -kephalin, natürliches Phosphatidylserin synthetisches 1-Palmitoyl-2-oleoylphosphatidylserin, Dimyristoyl- oder Dipalmitoylphosphatidylserin oder natürliche Phophatidsäure homogen mischt und dispergiert.

10. Verfahren nach einem der Ansprüche 1-9, dadurch gekennzeichnet, dass man die homogene Mischung durch Lyophilisat- oder Filmbildung herstellt.

11. Pharmazeutische Zusammensetzung in Form eines Verabreichungssystems auf Liposomenbasis für verkapselte Wirkstoffe herstellbar nach dem Verfahren gemäss Anspruch 1 gegebenenfalls vermischt mit physiologisch verträglichen Zusatzstoffen.

12. Pharmazeutische Zusammensetzung gemäss Anspruch 11 zur Anwendung bei der Behandlung des menschlichen oder tierischen Körpers.

FO 7.4 RS/we*